# EUROPEAN PATENT APPLICATION

(11) **EP 2 681 996 A1**
(43) Date of publication of application: **08.01.2014**
(21) Application number: 12174837.0
(22) Date of filing: 03.07.2012
(51) Int. Cl.: A01N 43/16, A61K 8/49, A61K 31/352, A01N 65/36, A01P 1/00

(54) **Biocidal compositions**

(71) Applicant: CeBeC Group Ltd., Galway (IE)
(72) Inventor: Daly, Sean, Co. Mayo (IE)
(74) Representative: Gates, Marie Christina Esther

(57) **Abstract**

A process for preparing a biocidal composition comprising the steps of: (i) dissolving at least one polyol in water to form a water/polyol co-solvent solution; (ii) adding at least one organic acid to the water/polyol co-solvent solution; (iii) adding at least one stabiliser to the water/polyol co-solvent solution; (iv) adding a flavonoid composition comprising at least one flavonoid selected from the group consisting of: poncirin, neoeriocitrin, isonaringin,rhoiflin, naringen, neodiosmin, hesperidin, neohesperidin and naringenin.
A non-microbial non-toxic biocidal composition having a pH in the range of 1 to 2.9, comprising at least one water soluble flavonoid compound, at least one organic acid, at least one polyol, at least one stabilizer, and water.

## Description

### Field of the Invention

The invention relates to biocial compositions derived from natural products and uses thereof.

### Background to the Invention

Biocides are used in the food industry, public & private health sectors, animal health, human and animal hygiene and wound care products to reduce or eliminate pathogenic microorganisms to decrease the likelihood of human or animal exposure to such infectious agents. Biocide use in the control of zoonotic pathogens has increased following an EU-wide ban (in 2006) on the use of preservatives and synthetic antimicrobial compounds as growth promoters in animal feed and the decline in available biocides under the EU Biocide Product Registry. Low concentrations biocides often act bacteriostatically and are only bacteriocidal at higher concentrations. Susceptible bacterial isolates acquire increased tolerance to biocides (biocide resistance) following serial transfer in growth media containing sub-inhibitory concentrations. Biocides displaying some bacterial resistance include QACs, bisbiguanides, biamidines, bisphenol, and acridines. Recently the emergence of biocide tolerant bacteria has been reported. Increasing tolerance to commonly used biocides may have an impact on a bacteriums response to food processing stresses such as heat, pH and others which are designed to reduce the risk to the consumer. Such a development may also enhance the virulence of particular zoonotic bacterial strains or may contribute to the enhancement of resistance to antimicrobial compounds, particularly those of critical importance to human health, such as the cephalosporins, fluoroquinolones and macrolides. Such zoonotic pathogens may exhibit an enhanced persistence and dissemination capacity *via* the food chain thereby posing an increased risk to consumer health. Current biocides range from chemicals such as alcohols, chlorine and its derivatives to ozone, hypochlorite and QACs. Each has associated positive and negative effects, including toxic by-products, poor biofilm penetration, corrosively, instability etc. Many are inherently unsuitable for certain applications, particularly so in the case of food production & public healthcare. Accordingly, new biocide products that demonstrate high target efficacy, with a low risk non-target toxicity and limited direct and antibiotic resistance development are required. Phytochemicals can have antimicrobial properties, but large-scale production ensuring consistent quality and efficacy at a manageable cost, along with formulations that permits diverse delivery and supports parallel mechanisms represents a technical and commercial challenge. Furthermore, phytochemicals generally suffer from incomplete characterisation and analysis, and potential non-sustainable responses. Plant antimicrobials are indigenously generated to manage bacteria and provide an evolutionary history and evidence of effective biocidal properties. For example, curromycins, cinnabarimides and oxazolomycins are also natural products with strong antibacterial activity. Another important class are plant polyphenols, which are secondary metabolites that can range from simple molecules, such as phenolic acids, to highly polymerized constituents such as tannins. Polyphenol derivatives in the plant kingdom are diverse, from fungi to *Angiospermae,* including food plants, and are common components in the human diet. All the phenolics, but especially flavonoids, have been reported to have multiple biological effects such as antioxidant activity, anti-inflammatory action, inhibition of platelet aggregation, inhibition of mast cell histamine release, and antimicrobial activities. Phenolic flavonoids may employ a number of antimicrobial mechanisms, but are largely considered as membrane-active 'biocides' by which, they moderate the cell wall, penetrate the cell, and can react with the cytoplasm and cellular proteins. Phenolic compounds are optimally biocidal active in an undissociated state in acidic and neutral media.

### Object of the Invention

It is an object of the invention to provide a low cost, highly active natural product based biocidal formulation with low non-microbial toxicity, yet high biocidial activity against a wide range of microbes including bacteria, biofilms, fungi, spores, viruses, and particularly against gut flora, etc., for use in a wide range of applications in various environments. It is a further object of the invention to provide such formulations having anti-viral, fungal or mycoplasma properties. It is a further object of the invention to provide a biocide formulation that has low risk of development of biocide resistance and secondary antibiotic resistance.

### Summary of the Invention

In one aspect of the invention, there is provided a process for preparing a non-microbial non toxic biocidal composition concentrate (designated hereafter as "C-Activ-NF") comprising the steps of:
(i) dissolving at least one polyol in water to form a water/polyol co-solvent solution;
(ii) adding at least one organic acid to the water/polyol co-solvent solution, adding each organic acid sequentially, if more than one organic acid is used;
(iii) adding at least one stabiliser to the water/polyol co-solvent solution; and
(iv) adding at least one water soluble flavonoid or at least one aqueous plant and/or fruit flavonoid extract, to form a non-microbe non-toxic biocide concentrate.

Suitably, the process steps are carried out sequentially, and in the order as described above. Preferably, the solutions formed are stirred after each addition until all powders are dissolved. To optimise and maximise dilution, mixing is preferably carried out by a rotator mixing process. Suitably, a minimum 20 RPM's (rotations per minute) is used, with optimum blending occurring typically between 60 and 80 rpm. Such speeds optimise and maximise dilution efficiency and prevent dissaption of critical ingredients. Low speed (60 to 80 rpm) mixing ensures that aeration and flocculation are minimised. Desirably, the step of dissolving at least one polyol in water to form a water/polyol co-solvent solution is carried out at a range of from about 45°C to about 55°C, preferably about 50°C. Desirably still, the step of adding at least one organic acid to the water/polyol co-solvent solution is carried out at range of from about 45°C to about 55°C, preferably about 50°C. Further desirably, the step of adding the flavonoid or flavonoid extract is carried out at range of from about 45°C to about 55°C, preferably about 50°C. Preferably, stirring occurs for typical processing time of from about 8 hours to about 24 hours per 1,000litre/kg to 10,000 litre/kg blends. The latter is dependent on an appropriate processing vessel. Dissolution of at least one polyol in water to form a water/polyol co-solvent solution is carried out at range of from about 45°C - from about 55°C, preferably about 50°C while addition of organic acids to the water/polyol co-solvent solution is carried out at about range of from about 45°C - from about 55°C, preferably about 50°C. Furthermore, the step of adding flavonoid is conducted at about a range of from about 45°C - from about 55°C, preferably about 50°C.

The components are added in the quantities defined below, such that the final pH of the composition ranges are preferably from about 1.0 to about 7.0, preferably from about 1.0 to about 6.0, preferably from about 3.0 to about 5.0, preferably from about 3.0 to about 5.0, but most preferably from about 1.0 to about 2.9, more preferably still from about 1.0 to about 2.0, depending on the degree of dilution. Preferably, the final pH of the concentrate (NF) ranges from about 1.0 to less than 3, preferably from about 1.3 to less than 2, preferably from about 1.5 to about 1.9, preferably from about 1.6 to about 1.8. The more concentrate the composition, the lower the pH. In some embodiments, it is preferedthat the mixture is not neutralized, as a highly acidic composition is preferred. In certain applications, neutralisation has been shown not to affect the activity and so slightly acidic to neutral solutions as described above can be used. In the concentrate, the flavonoid concentration (or flavonoid extract concentration) when measured by UV, is preferably greater than 2.5% w/w., more preferably is about from 3 to 12%, more p prefereably is from about 4 to 10%, and most preferably still from about 4.5 to 6.5, most preferably is 5%w/w. The total acids content is preferably greater than 35% w/w. The polyol content is preferably less than 25% w/w. Suitably, the water content is preferably less than 35% w/w. Preferably, the concentrate has a specific gravity at 20°C of from about 1.20 to about 1.25. Preferably, the viscosity of the concentrate at 20°C of from about 20 to about 100cP. The process provides a biocial composition concentrate ("C-Activ-NF"), which is easy to store, ready for direct use, or in diluted form, or as an active substance for inclusion in commercial products, such as hygiene products, foods, animal feeds etc. The concentrate itself, and active dilutions (working solutions) of the concentrate (hereinafter designated as "C-Activ-12") have unexpectedly good biocide activity against both positive and negative gram bacterial, viruses, fungi, fungal spores and even biofilms.

In a second aspect of the invention, an active working solution of the biocidal composition may be prepared by:
(i) dissolving at least one organic acid in water to form a 10% w/w solution of organic acid;
(ii) dissolving a biocidal composition concentrate (C-Activ-NF) into the solution of step (i);
(iii) diluting the solution formed in step (ii) with water,
to form an active working solution of a non-microbial non toxic biocidal composition comprising a 1/10 concentration of the biocidal composition (NF).

Suitably, the process steps are carried out sequentially.

Preferably, the organic acid is added at a concentration of about 8 - 12% w/w, preferably 10% w/w of the active biocidal solution. Preferably, the organic acid is citric acid. Preferably, the biocidal composition concentrate is added at a concentration of about 8 - 15% w/w, preferably about 10 - 12% w/w, preferably about 10.1 - 12% w/w, preferably about 10.1 - 11% w/w, and most preferably at 10% w/w of the active biocidal solution. The remaining component (80% w/w) is water. Preferably, the optimum active biocide composition has about 10% concentrate, about 10% citric acid and about 80% water. The pH of the active working solution is preferably from about 2.0 to about 7, about 2.0 to about 3.5, preferably from about 2.0 to less than 3.0, preferably from about 2.0 to about 2.9, or from about 2.0 to about 2.8, from about 2.0 to about 2.5, from about 2.2 to about 2.5. In one embodiment, where neutralisation occurs, the pH may be about 3 to about 7.In a preferred embodiment, a 5% aqueous solution of the working solution may have a pH of from about 2.0 to less than 3.0, preferably from about 2.0 to about 2.9, or from about 2.0 to about 2.8, from about 2.0 to about 2.5, from about 2.2 to about 2.5.

In a related aspect, there is provided a composition suitable for direct use as a biocide (concentrate: C-Activ-NF) or for use in preparing an active working biocide composition (working solution C-activ-12) comprising:
at least one water soluble flavonoid compound, optionally in the form of an aqueous fruit or plant flavonoid extract;
at least one organic acid;
at least one polyol; and
at least one stablizer; and
the remainder of the composition is water.

In a preferred embodiment, there is provided a non-microbial non-toxic biocidal composition, having a pH in the range of from about 1 to 2.9, more preferably from about 1 to 2, comprising:
from about 0.1% to about 15% w/w of at least one water soluble flavonoid compound, optionally in the form of an aqueous plant part extract comprising at least one flavonoid;
from about 8% w/w to about 12% w/w of at least one organic acid;
from about 30% w/w to about 50% w/w of at least one polyol; and
from about 5% w/w to about 10% w/w of at least one stablizer; and
the remainder of the composition is water.

Preferably, the composition has a pH in the range of from 1.0 to 2.0. When the former composition is diluted (1/10) to provide the later active working biocide composition, the active working composition has a pH in the range described above.

Accordingly, in a preferred embodiment, there is provided a working active composition, wherein the composition concentrate above is diluted with water/organic acid solution, and comprises:
from about 0.01% to about 1.5% w/w of at least one water soluble flavonoid compound, optionally in the form of an aqueous plant part extract comprising at least one flavonoid;
from about 11.5% w/w to about 22.5% w/w of a first organic acid;
from about 1.5% w/w to about 2.5% w/w of a second organic acid;
from about 1% w/w to about 1.5% w/w of at least one polyol; and
from about 0.5% w/w to about 1% w/w of at least one stablizer; and
the remainder of the composition is water.

Preferably, each of the biocial composition concentrate "C-Activ-NF" and the active diluted biocidal composition "C-Activ-12" comprise at least one flavonoid selected from: poncirin, neoeriocitrin, isonaringin, rhoiflin, naringen, neodiosmin, hesperidin, neohesperidin or naringenin. Suitably, a mixture of two or more flavonoids selected from: poncirin, neoeriocitrin, isonaringin, rhoiflin, naringen, neodiosmin, hesperidin, neohesperidin and naringenin are present. In a particularly preferred embodiment, the composition comprises at least poncirin, neoeriocitrin, isonaringin, rhoiflin, naringen, neodiosmin, hesperidin, neohesperidin and naringenin. The flavonoids may be added in substantially pure chemical form, or may be added as part of a natural composition or an aqueous plant extract comprising one or more of the water soluble flavonoids. Preferably, such flavonoid extract may be obtained from an aqueous extract of citrus aurantium/bitter oranges, Seville oranges, Citrus paradise and/or grapefruit. More preferably still, the extract may be from the pulp of fruit, the leaves, the flowers and/or the seeds of plants.

Preferably, the total flavonoid concentration (either directly added flavonoids or a plant extract containing flavonoids) is in the range of from about 0.1% to about 15% w/w based on the total weight of the concentrate composition (NF), preferably from about 1% to about 15% w/w, preferably from 3% to about 13% w/w, preferably from about 5% to 11% w/w, preferably from about 6% to 10% w/w, and most preferably from 10% to 12% w/w based on the total weight of the composition. In one preferred example, the total flavonoid concentration is about 2.25% w/w of the concentrate, where the flavonoids are provided in the form of 5% w/w based on the total weight of the composition of a component being a suitable aqueous plant and/or fruit extract of the concentrate (the remainer of the extract is typically biomass). In one particularly preferred embodiment, the flavonoid is present in the biocide composition as a suitable aqueous plant and/or fruit extract from about 5% to about 12% w/w in the concentrate (C-Activ-NF), and at about 0.5% to about 1.2% w/w in the working composition (C-Activ-12), and from about 0.0025 - about 0.012% w/w in the final working solutions based on recommended working concentrations.

Preferably, at least two flavonoids are included. Suitably, at least naringen and neohesperidin are included. Where naringen and neohesperidin are present, their total concentration preferably makes up less than 75%, more preferably less than 70%, preferably less than 60%, preferably less than 50%, preferably less than 40%, and most preferably less than 35% of the total bioflavonoids present in the composition. Preferably, the flavonoid components make up preferably from less than 30% w/w, more preferably from less than 15% w/w of the aqueous flavonoid extract used (when other plant components -biomass is considered). Preferably, at least three flavonoids are included. For example, where the flavonoid isonaringin is also included in the composition, the isonaringin concentration is preferably in excess of 10%, in excess of 15%, excess of 20% in excess of 35%, and most preferably in excess of 45%, but in any case less than 60%. Preferably, this component make up greater than 5% w/w, more preferably greater than 15% w/w of the aqueous flavonoid extract used (when other plant components -biomass as considered).

In a particularly preferred embodiment, there is provided a composition suitable for use as a biocide comprising:
(i) at least two water soluable flavonoid compounds, optionally in the form of a flavonoid aqueous extract or composition comprising at least two water soluble flavonoids, wherein the total flavonoid concentration is in the range of from about 0.1% to about 15% w/w based on the total weight of the composition, and wherein less than 75% of the bioflavonoids are naringen and neohesperidin;
(ii) at least one organic acid;
(iii) at least one polyol; and
(iv) at least one stablizer; and
(v) the remainder of the composition is water.

Preferably, the composition has a pH in the range as discussed above. Suitably, naringen and neohesperidin and isonaringin are present in the amount described above.

In a preferred embodiment, the polyol may be selected from: glycerine, ethylene glycol, sucrose or pentaerythritol, or mixtures thereof. The polyol component is preferably in the range of from about 15% w/w to about 25% w/w, more preferably from about 17% to about 23%, from preferably about 20% w/w to about 22% w/w, most preferably 21.5% w/w of the total composition.

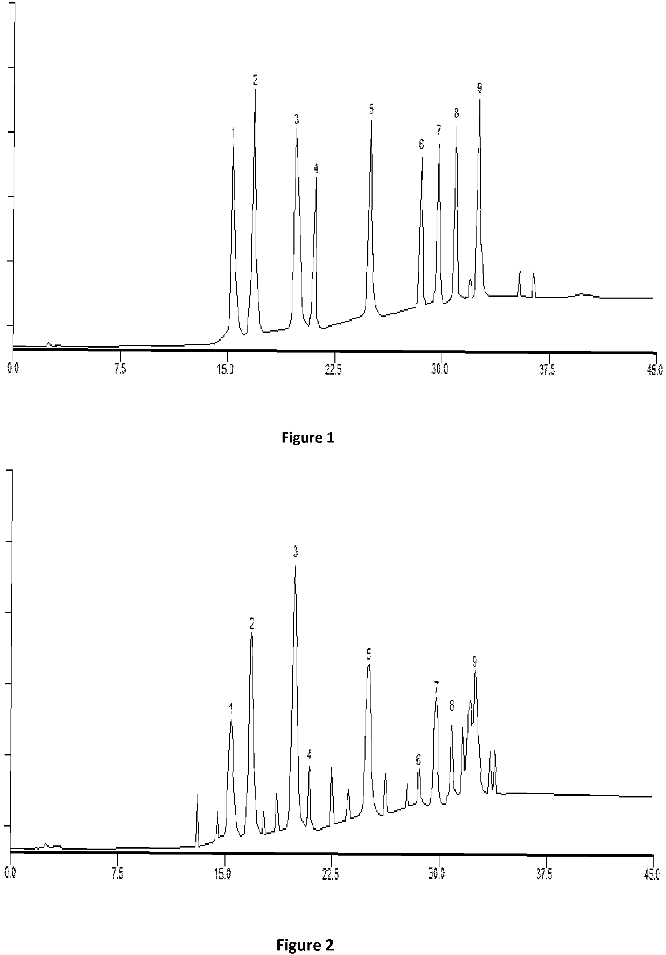

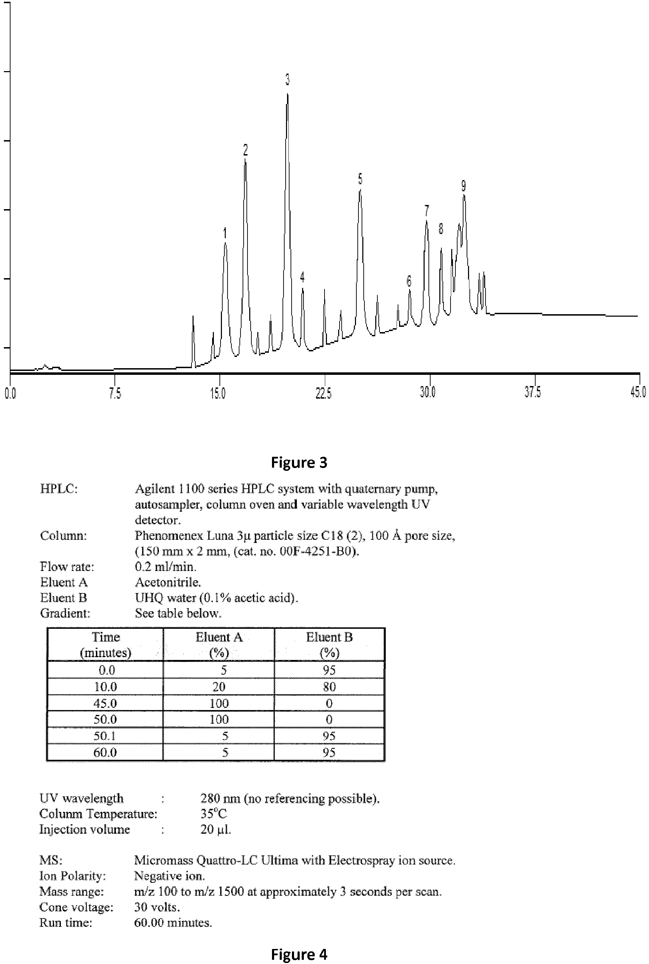

Glycerine is a particularly preferred polyol. Preferably, the ratio of water to polyol in the mixture is in the range of from about 1.3:1 (water: polyol) to about 2:1 (water: polyol), most preferably the range is about 1.4:1 (water: polyol), and most preferable still water: polyol are present in a ratio of about 1.44: 1. In a further preferred embodiment, the organic acid is a fruit acid selected from: lactic acids, acetic acids, malic acids or citric acids, or mixtures thereof. Preferably, at least two organic acids are present. Preferably, the ratio of acids is from about 0.5:1.5 to 1.5:0.5. Preferably, the ratio of acids is about 1:1. Desirably, two organic acids, are citric acid and malic acid. Preferably, the ratio of citric acid to malic acid is about 1:1. Suitably, the stabliser may be selected from: ascorbic acid, uric acid or polyphenol. In a preferred embodiment, the stabilizer is present in the range of from about 5% w/w to about 10% w/w, more preferably from about 6% to about 9%, from preferably about 7% w/w to about 8% w/w, most preferably 7.52% w/w of the total composition. Preferably, the ratio of stabilizer to organic acid in the composition is in the range of from about 1:2:2 (stabilizer: organic acid: organic acid - assuming more than one is used) to about 2.5:3.2:3.2, most preferably the range is about 1:2:2 (stabilizer: organic acid: organic acid), and most preferable still 1:2:2 (ascorbic acid: malic acid: citric acid). Preferably, ascorbic acid is used as the stabliser as it prevents oxidation of the flavonoids and accordingly increases the stability and performace of the biocide composition. In keeping with the safe and natural status of this product, only biosourced antioxidants components were employed. In a preferred embodiment, the biocide concentrate composition (C-Activ-NF) is diluted with water to a concentration of 1/10 w/w to provide an active working concentration. This concentration of product is suitable for the majority of biocide applications. The flavonoid profile of the composition typically may has the HPLC fingerprint substantially corresponding to that Figure 2 or 3, which measured at different concentrations. The flavonoid composition of C-Activ-NF has the HPLC fingerprint of Figure 2 (note - it is required to dilute C-Active-NF to attain the working concentration needed for the HPLC analysis). The working composition that comprises a 1/10 dilution of the biocide concentrate of the invention has a HPLC fingerprint substantially corresponding to that of Figure 3 (C-Activ-12). Typically the retention times and percentage of flavonoids present are evidenced by the HPLC in the concentrate under HPLC (conditions described below) comprise (a) Poncirin 15.9 minutes - 3.7% (b) Neoeriocitrin 16.5 minutes - 7.5% (c) Isonaringin 19.6 minutes - 38.5% (d)Rhoiflin 20.4 minutes - 6.6% (e) Naringen 24.1 minutes - 22.7% (f) Neodiosmin 28.4 minutes - 4.0% (g) Hesperidin 29.4 minutes - 5.2% (h) Neohesperidin 30.2 minutes - 4.6% (i) Naringenin 32.9 minutes - 7.2% Typically the retention times and percentage of flavonoids present are evidenced by the HPLC of a working solution of one exemplary formulation of C-Activ-12 under HPLC (conditions described below) comprise (a) Poncirin 15.9 minutes - 2.6% (b) Neoeriocitrin 16.5 minutes - 4.9% (c) Isonaringin 19.6 minutes - 37.7% (d)Rhoiflin 20.4 minutes - 24.4% (e) Naringen 24.1 minutes - 24.9% (f) Neodiosmin 28.4 minutes - 4.4% (g) Hesperidin 29.4 minutes - 6.0% (h) Neohesperidin 30.2 minutes - 7.8% (i) Naringenin 32.9 minutes - 7.3%

| **Dilution -ACTIVE WORKING (1/10) v/v** | **Range %w/w Ex1** | **Range %w/w Ex2** | **Example %w/w** |
|---|---|---|---|
| Organic acid | 8-12 | | 10 |
| NF | 8-15 | | 10 |
| Water | Balance | | Balance |
| **Total** | **100%** | | **100%** |
| pH | 2.1-2.6 | | 2.1-2.6 |

| **Flavonoids** | **Example % of Flavonoids** | | **% of Total w/w Flavonoid Extract** |
|---|---|---|---|
| Poncirin | 3.7 | | 1.7 |
| neoeriocitrin | 7.5 | | 3.4 |
| isonaringin | 38.5 | 56.6 | 17.3 |
| rhoiflin | 6.6 | | 3.0 |
| naringen | 22.7 | | 10.2 |
| neodiosmin | 4.0 | | 1.8 |
| hesperidin | 5.2 | 3.5 | 2.3 |
| neohesperidin | 4.6 | 6.75 | 2.1 |
| naringenin | 7.2 | 25.9 | 3.2 |
| Other plant components | - | | 55 |
| **Total** | **100** | | **100** |

For the most part, the flavonoid component is made up of mostly isonaringin, followed by naringen which preferably make up about half (about 50%) of the flavonoid component. The third preferred flavonoid is neohesperidin, although levels of same are only required from about 4% upwards. Other examples of the working active indicate isonaringen present at 56.5%, naringen at 25.9%, hesperiden at 3.5% and neohesperiden at 6.75%. Proportional composition has been found to be similar in concentrate and working active wherein total naringen and neohesperiden is about 33%.

| **NF Concentrate** | | | | | | | | |
|---|---|---|---|---|---|---|---|---|
| Flavonoid Ex | 5% w/w Total Composition with % w/w Flavonoid in Extract | | | | | | | |
| Flavonoid Content | 3-15 | 4-13 | | 5-11 | 6-10 | | 10 - 12 | 45 |
| *Naringen &* Neohesperidin | Maximum less than 75% of flavonoid total | | | | | | | |
| *Isonaringin* | Minimum in excess of 25% of flavonoid total | | | | | | | |

| | **Range %w/w** | **Range %w/w** | | **Range %w/w** | **Range %w/w** | | **%w/w** | **% (w/w)** |
|---|---|---|---|---|---|---|---|---|
| (Organic acids) | (30-40) | 15 - 17 | | 16 - 18 | 17 - 19 | | -18 - 20 | (35.04) |
| -malic | 15-20 | | | | | | | 17.52 |
| -citric | 15-20 | 15 - 17 | | 16 - 18 | 17 - 19 | | 18-20 | 17.52 |
| Polyol | 15-25 | 11-14 | | 20 - 22 | 13 - 14 | | 13.5 - 13.7 | 21.52 |
| Stabilizer | 5-10 | 6-9 | | 7-8 | 8-9 | | 9-10 | 7.52 |
| Water | Balance | Balance | | Balance | Balance | | Balance | Balance |
| Total | 100% | 100% | | 100% | 100% | | 100% | |
| pH | 1.0-2.0 | 1.0-2.0 | | 1.0-2.0 | 1.0-2.0 | | 1.0-2.0 | |

| Typical optimal % composition | | | | | | | | |
|---|---|---|---|---|---|---|---|---|
| | | | % per NF | | | % per E12 | | |
| **Component** | | | | | | | | |
| RO water | | | 31 | | | 83.1 | | |
| Polyol | | | 21.5 | | | 2.15 | | |
| Stabiliser | | | 7.5 | | | 0.75 | | |
| Citric Acid | | | 17.5 | | | 11.75 | | |
| Malic Acid | | | 17.5 | | | 1.75 | | |
| Flavonoid Extract | | | 5 | | | 0.5 | | |

The composition may further comprise at least one other components/adjuvant selected from surfactants, for example SDS, alcohols, coatings, bleach etc. The compositions may further comprise at least one acceptable carrier, preferably those safe for use in food and water applications. The composition of the invention may also be formulated with various delivery technologies to kill harmful organisms in the food sector from farm to fork, in human health and animal health, human and animal hygiene products, protective coatings and surface sanitisation. The product has also been successfully added to drinking water for poultry reducing loadings of harmful bacteria in the gut of the bird and is approved as a Potable water treatment, non-rinse surface sanitiser and food processing aid in Australia & New Zealand. The composition may be formulated for use in an aerosol dispenser, or with other liquids for disinfection, in the form of encapsulated granule, capsule suspension, emulsifiable concentrates, emulsions for seed treatment, flowable concentrate for seed treatment, cold or hot fogging concentrate, solution for seed treatment, suspension/flowable concentrate, soluble concentrate (see OECD monograph guidance March 2001 for more details).

The biocide composition C-Activ-12 prepared from the composition concentrate (C-Activ-NF) of the invention has been consistently found to be very active against both gram positive and gram-negative bacteria, and thus as a natural biocide represents a welcome alternative from existing available chemically synthesised biocides such as chlorine, ammonia, sodium hypochlorite, etc. The compositions find use as a biocide, as a sanitiser, in human or animal hygiene products/biocidal products, veterinary hygiene biocidal, as an antimicrobial or as a disinfectant against microbes, planktonics, fungi, fungal spores or biofilms. Further uses include a food sanitiser or a wash for food produce such as salads, vegetables, fruit and plants, poultry and meat carcasses, as a barrier protectant, as a poultry processing aid such as a wash or spray, a drinking water additive for animals, surface sterilizers or disinfectant for surface and equipment. In a further embodiment, the composition may be used in packaging for storage for extension of shelf life & growth inhibition. In a further embodiment still, the composition may be used in biosecurity & environmental management of microorganisms or in private area and public health areas disinfectants, as and as food and feed area disinfectants, as a drinking water disinfectant for human and animal consumption, or as a potable water treatment. Other uses include preservative applications such as preservatives for food or feedstocks, in-can preservatives, film preservatives/packaging, polymerised materials preservatives, preservatives for liquid-cooling and processing systems, crop and plant protection products. The can also be used as biocidal formulations that protect general hard surfaces and that has activity against antibiotic resistance bacteria for use in public health applications. The composition provide a biocidal formulation which has highly adaptive protective properties suitable for food applications (for example, processing & preparation and preservation) and ensure extended mechanisms of action rendering the formulations suitable for use on food, in food and on food production surfaces, processing surfaces, water treatment and other related food sector treatment applications. They can also be used to treat wounds and infections.

In a preferred embodiment the biocide is active against gram positive and/or gram negative bacteria. Activity is against at least one bacterial species selected from the group consisting of: *Salmonella,* Campylobacter, *Enterococcus, Listeria monocytogenes, Pseudomonas, Escherichia* and *Stapylococcus* and *Pseudomonas.* In a preferred embodiment the biocide is used against *at least one bacterial strain* selected from the group consisiting of: *Salmonella typhimurium, Campylobacter jejuni, Enterococcus hirae, Listeria monocytogenes, Pseudomonas aeruginosa, Escherichia coli* and *Stapylococcus aureus, C. sakazakii, S. pollorum, P. cangingivalis, P. Salivosa, F. nucleatum, Eikenella corrodens, Bacteroides fragilis, Prevotella intermedia, Porphyromonas gingivalis, Tanerrella forsythensis, Cronobacter sakazakii.* In a preferred embodiment the biocide is effective against fungi, powdery mildew, botrytis and Penicillium.

### Brief Description of the Drawings

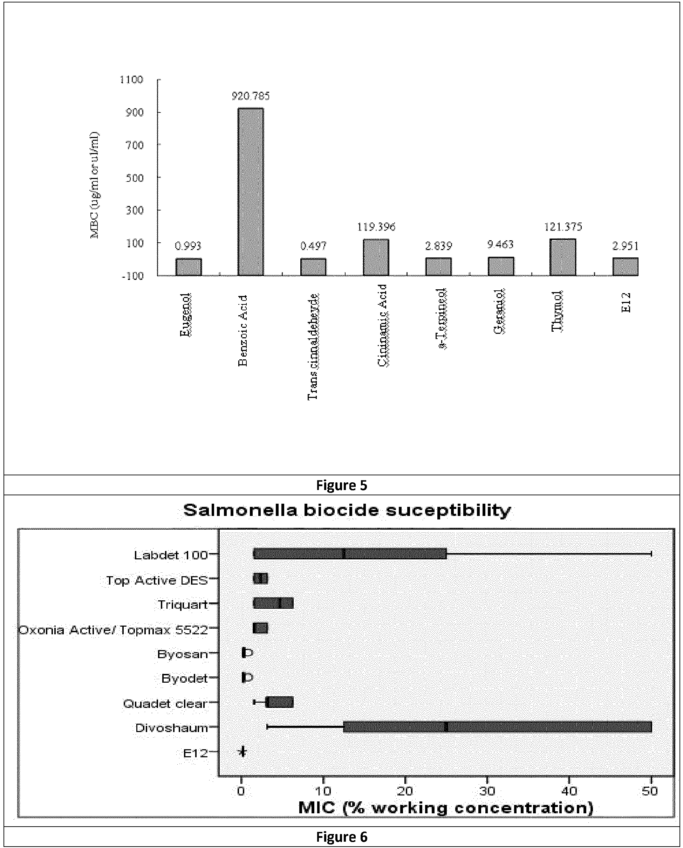

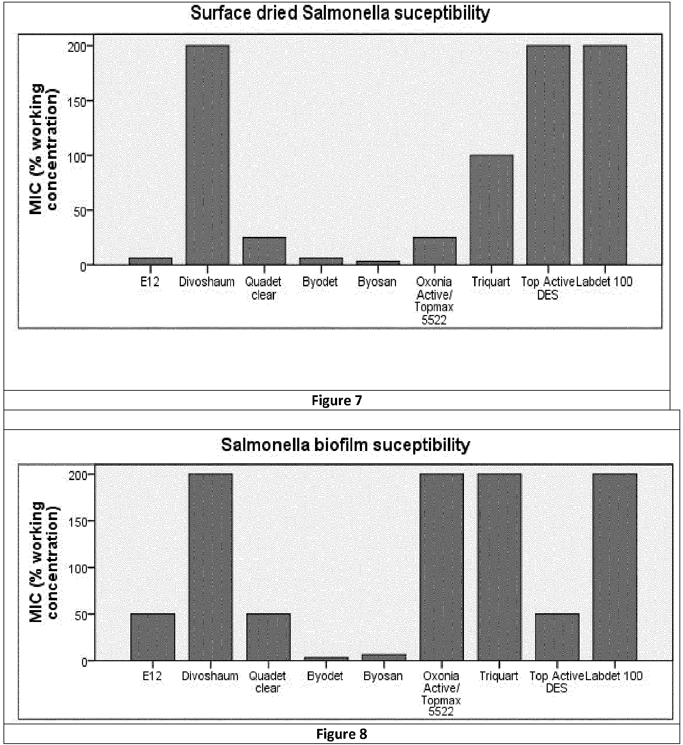

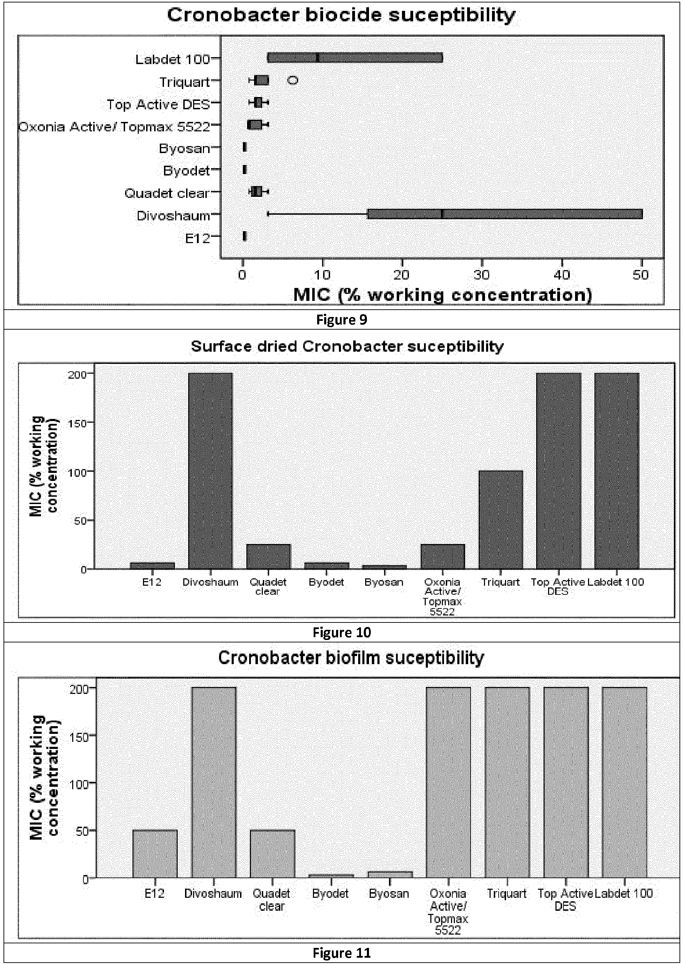

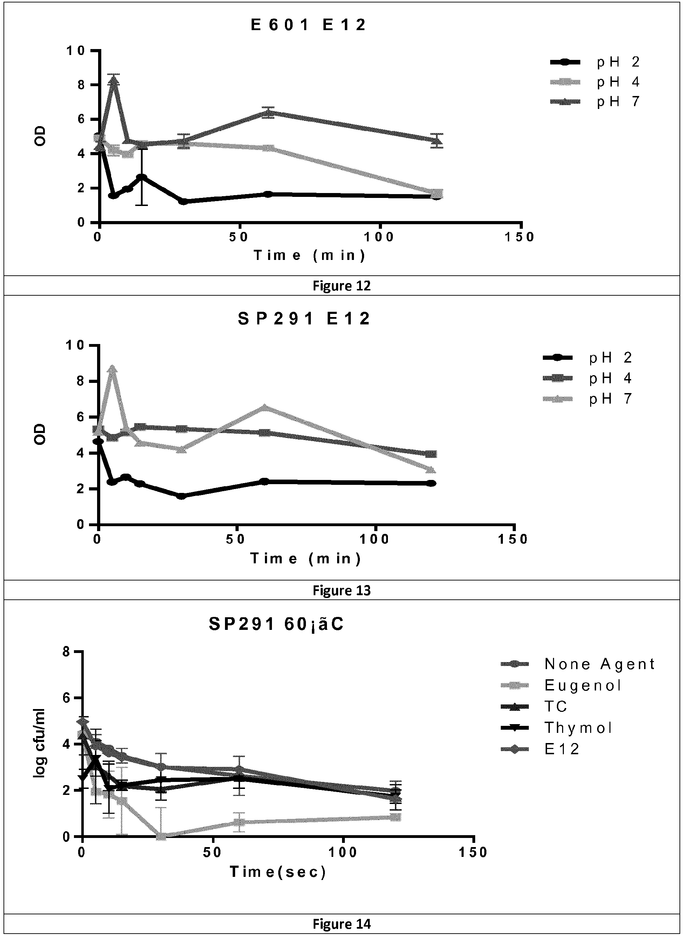

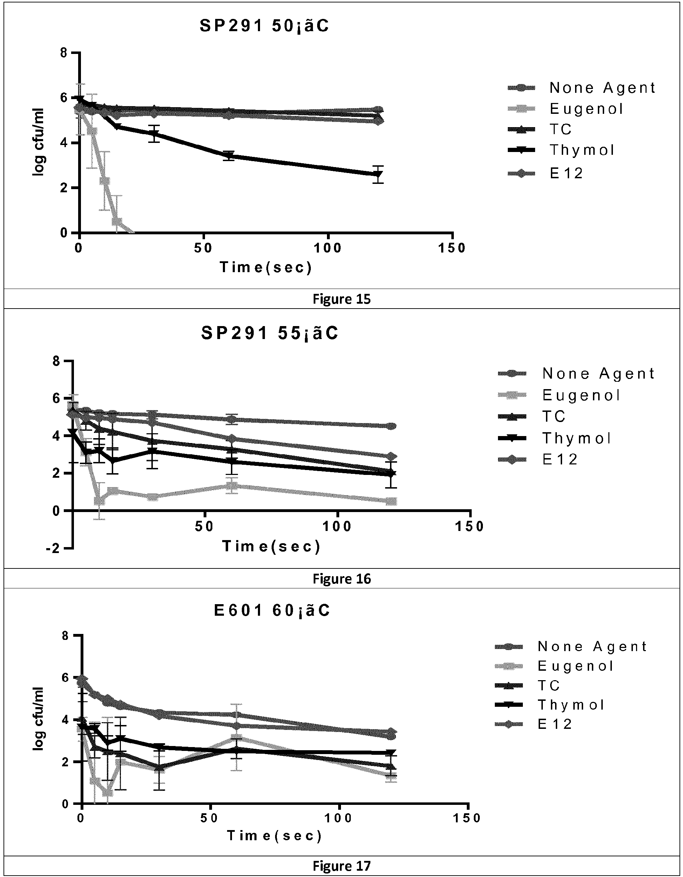

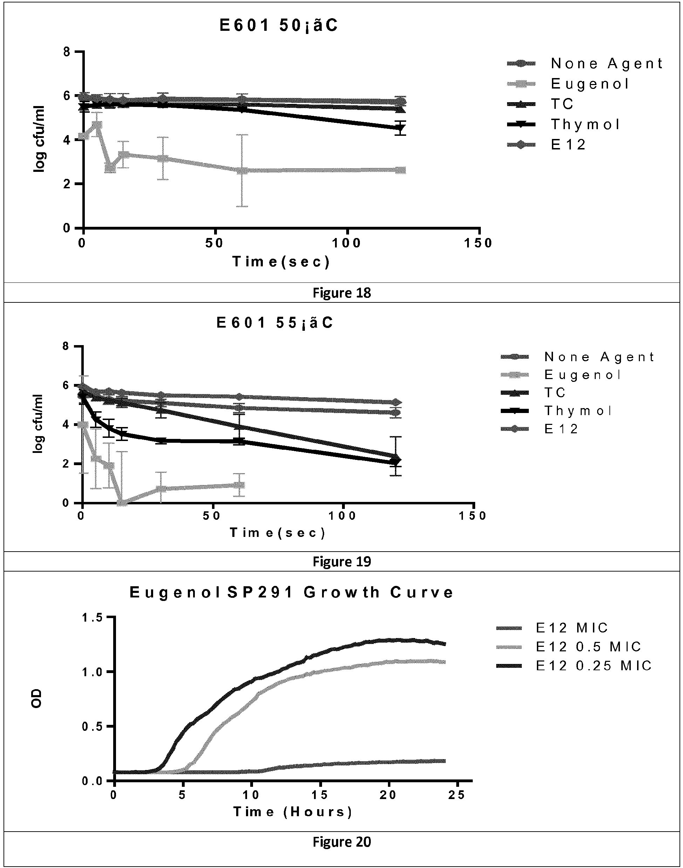

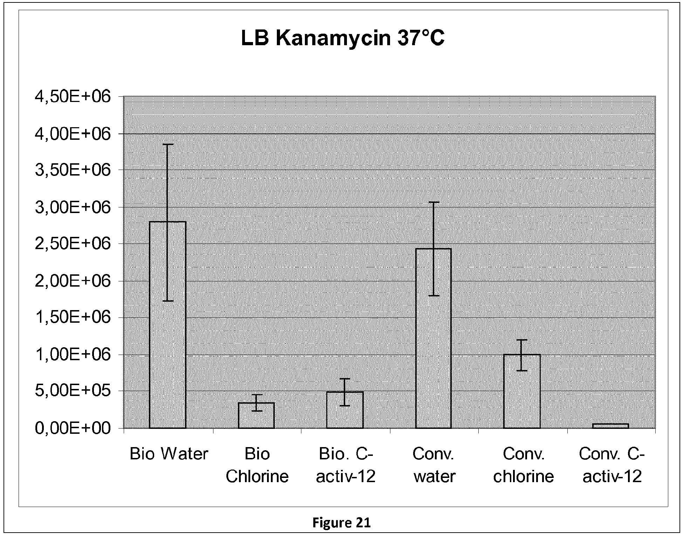

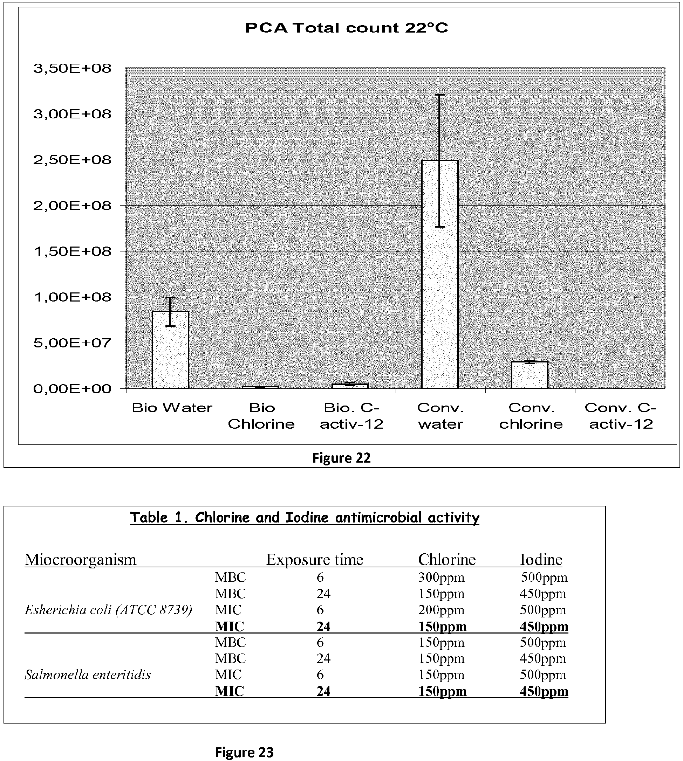

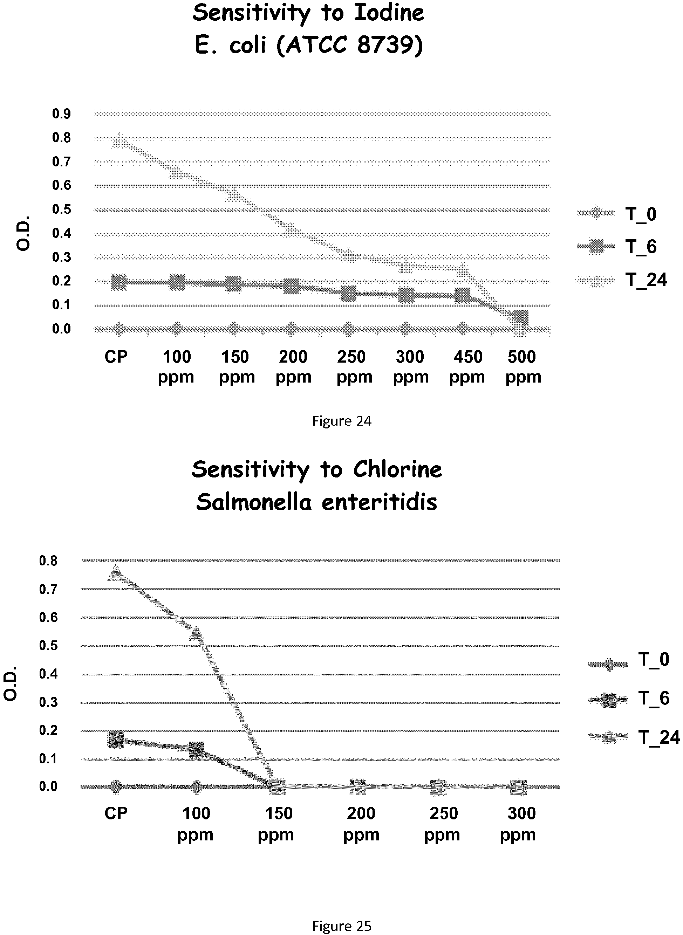

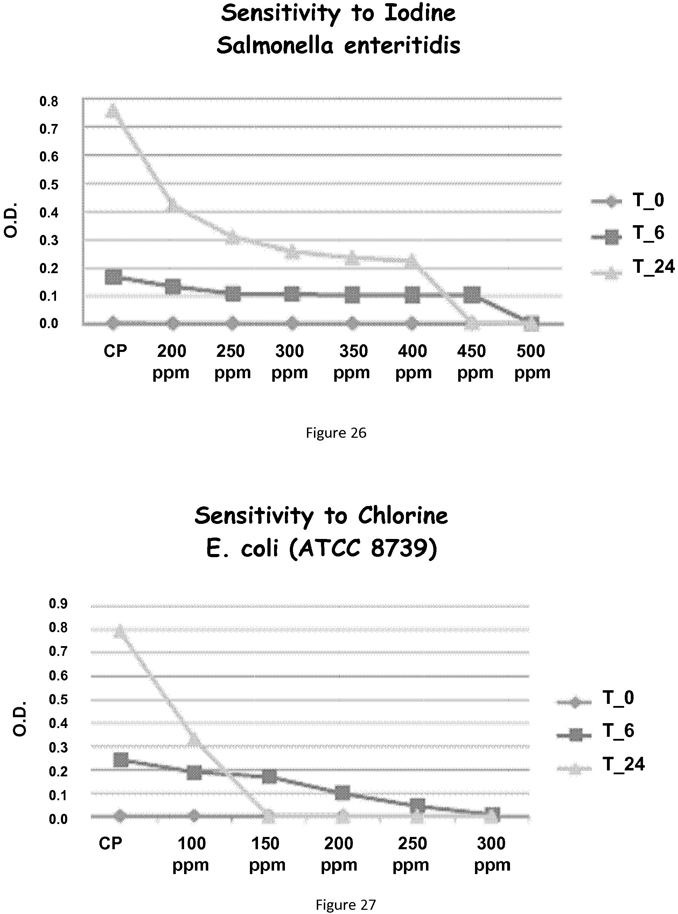

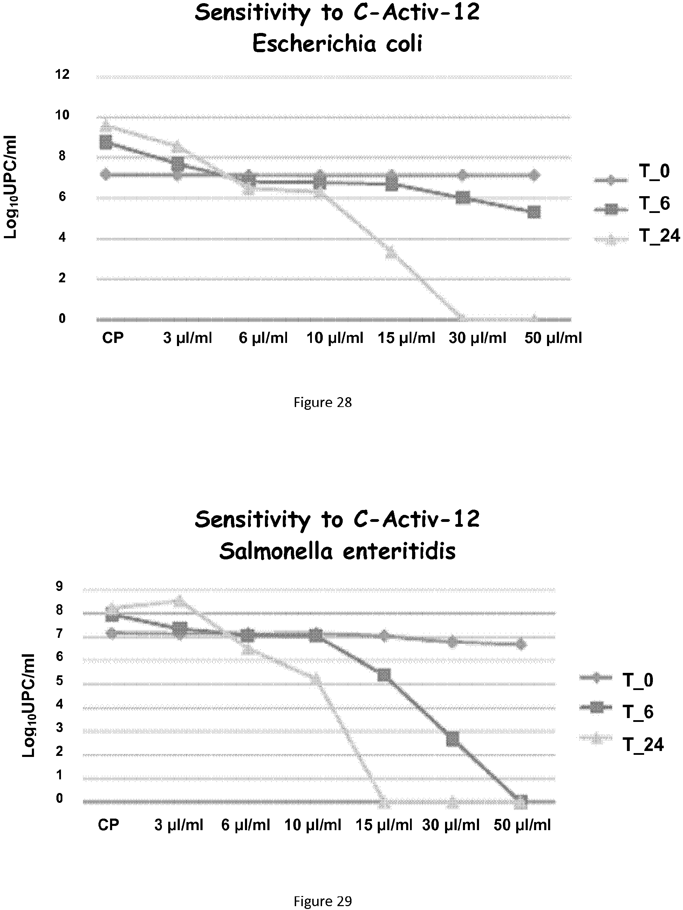

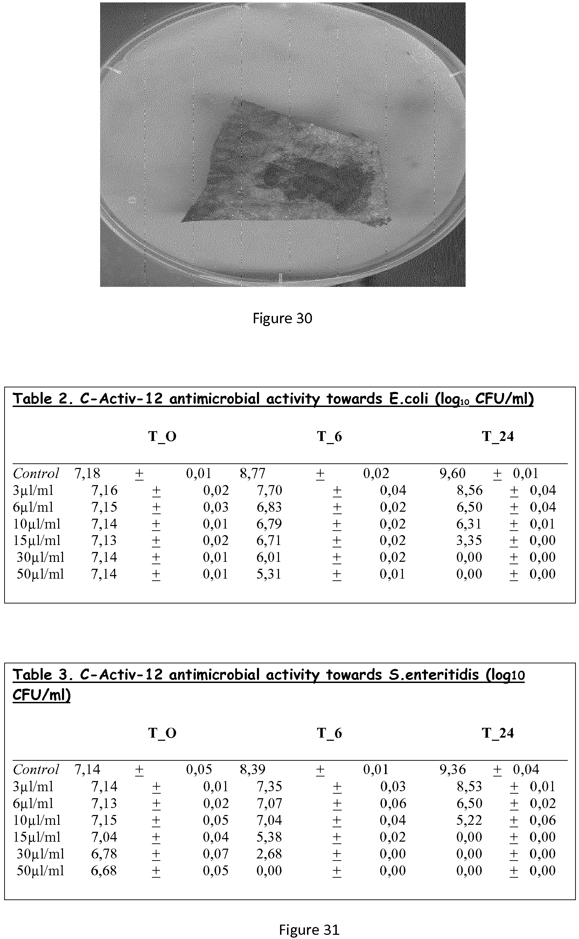

**Figure 1** illustrates a HPLC chromatogram of a standard solution of the bioflavonoid standard solution at 250ppm concentration; **Figure 2** illustrates a HPLC chromatogram of the *"C-Activ-NF"* biocide concentrate composition of the invention at a 1/1000 dilution; **Figure 3** illustrates a HPLC chromatogram of the *"CeBeC Active 12 (C-Activ-12)"*) biocide composition of the invention at a 1/100 dilution; Figure 4 illustrate typical HPLC conditions used for analysis. **Figure 5****:** Replicate MIC analysis of E12 relative to other antimicrobials; **Figure 6****:** Salmonella Biocide Susceptibility (8 biocides relative to C-Activ-12); **Figure 7****:** Surface Dried Salmonella Susceptibility (8 biocides relative to C-Activ-12); **Figure 8****:** Salmonella Biofilm Susceptibility; **Figure 9****:** Cronobacter biocide susceptibility; **Figure 10****:** Surface dried Cronobacter susceptibility; **Figure 11****:** Cronobacter biofilm susceptibility; **Figure 12****:** pH Tolerance C-Activ-12 (E12); **Figure 13****:** pH Tolerance C-Activ-12 (E12) - replicate; **Figure 14****:** Heat Tolerance of C-Activ-12 (E12) when compared to other Active Substances/Essential Oils; **Figure 15****:** Heat Tolerance of C-Activ-12 (E12) when compared to other Active Substances/Essential Oils; **Figure 16****:** Heat Tolerance of C-Activ-12 (E12) when compared to other Active Substances/Essential Oils; **Figure 17****:** Heat Tolerance of C-Activ-12 (E12) when compared to other Active Substances/Essential Oils; **Figure 18****:** Heat Tolerance of C-Activ-12 (E12) when compared to other Active Substances/Essential Oils; **Figure 19****:** Heat Tolerance of C-Activ-12 (E12) when compared to other Active Substances/Essential Oils; **Figure 20****:** E. coli growth curve in the presence of C-Activ-12 and eugenol (phenyl propanoid); **Figure 21****:** Biological & conventional salad exposed to Kanamycin resistant E. coli - C-Activ-12 showed highest log reduction; **Figure 22****:** Biological & conventional Salad subjecto Wash Plate Count Agar - C-Activ 12 showed highest log reduction; **Figure 23****:** Cl and I antimicrobial activity; **Figure 24****:** Sensitivity to iodine *(E. coli*); **Figure 25****:** Sensitivity to chlorine *(S. enteritidis);* **Figure 26****:** Sensitivity to iodine *(S. enteritidis);* **Figure 27****:** Sensitivity to sodium hypochlorite *(E. coli*); **Figure 28****:** Sensitivity to C-activ-12 (*E.coli*); **Figure 29****:** Sensitivity to C-Activ-12 (S. *enteritidis);* **Figure 30****:** Powdery mildew *(Uncinula necator)* on cucumber leaf in the presence of a 300 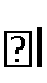 l of 2% C-Activ-12. C-Activ-12 inhibited fungal activity; **Figure 31****:** Antimicrobial activity

### Detailed Description of the Invention

The Cebec research, formulation work and initial testing that was performed, which led to and drove the development of this new biocide product, C-Activ-NF & C-Activ-12, were conducted in 2009. Abbreviations - ATCC = American Type Culture Collection, BSA = Bovine Serum Albumin, BSEN = British Standard European Norm, CFU = Colony Forming Units, CNMR = Centre for Nanotechnology and Materials Research, E12 = Laboratory code for C-Activ-12, FDA = Food & Drug Administration, GRAS = Generally Recognised As Safe, MIC = Minimum Inhibitory Concentration, mM = milliMolar, NaCl = Sodium chloride, NCTC = National Collection of Type Cultures, °C = degree celsius, ppm = parts per million, spp = species, TNTC = To Numerous To Count, TSA = Tryptic Soy Agar, v/v = volume / volume, w/v = weight / volumn The Inventors have developed a stable, highly active natural product biocide composition concentrate known as C-Activ-NF and a working concentrate solution known as C-Activ-12. The preferred biocide composition concentrate (C-Activ-NF) of the invention consists of at least one natural organic acids, (for example, malic acid, citric acid), at least one polyol, (for example glycerine), and at least one antioxidant, (for example ascorbic acid), together one or more flavonoid compounds (for example, hesperidin, naringin, isonaringin, etc.) or a flavonoid extract from a suitable source such as one or more of citrus aurantium/bitter oranges, Seville oranges, Citrus paradise and or grapefruit, and particularly, extracts from the pulp of fruit, the leaves, the flowers and seeds of plants. The flavonoids were extracted from selected citrus fruits of a particular age range and time of year, following physical disruption, by an aqueous water/methanol/ethanol solvent mix. No solvent residue was retained in final lyophilised product. Preferably, the extracts are aqueous extracts as the flavonoids of interest are a class of water-soluble active phytochemicals. Flavonoids and flavonones levels, are influenced by growing and environmental factors. Fruit sourcing factors are important in determining variations in the levels of concentration of these compounds, the quantity in immature bitter orange fruit being higher than in ripe fruit for example. The bitter orange extracts can be obtained from ground citrus fruits (especially Citrus aurantium/bitter oranges) through standardized processing operations such as extraction, filtration, concentration, precipitation, clarification and final drying. Extraction processes can be performed in cold water extraction systems, or wherein a solvent is selected from methanol, ethanol, propanol and the like. Methanol is used preferably, but not exclusively, and as such any solvent used must comply with EU Council Directive 88/344/EEC of 13 June 1988 on the approximation of the laws of the Member States on extraction solvents used in the production of foodstuffs and food ingredients. The compositions of the invention were analysed using HPLC against a series of known biofavonoid standards. A HPLC chromatogram of the standard solution used (at a concentration of 250ppm) for fingerprinting and quality control is shown in the Figures. Raw material sourcing and processing described herein generates a stable biocial composition that displays unique and consistent biocidal properties, even at very low concentrations, when subject to multiple comparative assay studies. The component concentrations employed, the addition sequence, the dilution measurement techniques, the solvent volumes, the C-Activ-NF concentrate, the C-Activ-12 Active Substance working biocidal formulation, the operational dilution, were all optmised by application of multivariable formulation studies. The flavonoid components of the compositions of the invention were selected on preliminary anti-oxidant evidence, commercial viability, cost and availability. Single or multiple flavonoids components can be present in the biocide compositions of the invention. Suitably, at least naringin, isonaringin and neohesperidin are incorporated. Food grade quality is necessary for the proposed applications (such products must comply with Council Directive 78/663/EEC which specifies the standards of purity for emulsifiers, stabilizers, thickeners, and gelling agents for use in foods). Organic acids such as lactic, acetic, malic, and citric acid are included in the composition of many foods for preservation purposes. Their ction on flavonoids is related to pH reduction, impediment of substrate transport, and reduction of proton motive force. The lower MW of malic acid ensures greater permeability than citric acid, but both were selected as the organic acid additives in the preferred compositions of the present invention. Flavonoids are water soluble, but at higher concentrations require incorporation of an organic solvent. The polyol glycerine is highly stable, virtually nontoxic and a non-irritant and odourless. Due to its hydroxyl groups, glycerine has solubility characteristics similar to those of water and simple aliphatic alcohols. Phenolic compounds are more soluble in glycerine than in water, and this additive was therefore selected to enhance flavonoid solubility and stability. While, free radicals may exert antimicrobial effects, they are equally likely to be cytotoxic to mammalian cells (if not locally controlled). Flavonoid stability will also be dependent on prevention of oxidation and so anti-oxidant, such as ascorbic acid was included in the formulation. This is also a common food anti-oxidant ingredient, with low secondary toxicity. Biocidal composition concentrates were prepared and sequential dilution protocols generated, but the majority of the formulation research was devoted to identifying optimal component concentrations for biocidal efficacy, requiring dilution sequences, mixing sequence and stirring duration and temperature study. The combination of starting materials and process parameters imparts unexpected antimicrobial activity to the compositions produced by the processes of the invention. The working biocidal formulation C-Activ-12 can be produced from the composition concentrate (C-Activ-NF) as described herein or it can be produced separately by adding the individual ingredients sequentially in one blending process under process blend protocols, stirring, temperature, solubility, timing, as described herein

### Preferred Biocial Composition Concentrate (C-Activ-NF) Blending Protocol for Biocidal Active

***Substance C-Activ-12-*** Most flavonoids exhibit low solubility and poor stability in both polar and nonpolar media, impeding incorporation in many formulations. The flavonoids employed herein were subject to a certain process to enhance dissolution and to provide viable and stable concentrations. The biocidal compositions of the invention are first blended as a concentrate (C-Activ-NF) for preparation of biocide, which can then be suitably diluted with an appropriate solvent, such as a composite of water and/or citric acid to generate the CeBeC Active 12 (C-Activ-12) Active Substance working biocidal composition. Typically, a dilution of 10 x of the concentrate composition C-Activ-NF produces a good working solution which has been shown to have excellent biocidal activity. The working concentrate can be subjected to further selected aqueous dilution for real time activity and exposure, again, depending on the nature of the application and susceptibility of the target. In a particularly preferred embodiment, the flavonone component of the biocidal composition concentrate (C-Activ-NF) of the invention is a suitable plant part extract and is present at about 5% - about 12% (w/v) in the concentrate (C-Activ-NF), about 0.5% - about 1.2% (w/v) in C-activ-12 working composition solution, and about 0.0025 - about 0.012% (w/v) in the final recommended dilutions/active concentration used in many of the tests presented herein. Examples of HPLC chromatogram of characterisation compliant products with are shown in Figures 2 & 3, C-Activ-NF & C-Activ-12. All components are dissolved sequentially and added in singular addition. Sequential addition, timing and mixer conditions maximises solubility. Preferred conditions were established following the trial development of the formulation. To optimise and maximise dilution, the rotator mixing process rotates at a minimum of about 20 RPM's (rotations per minute), with optimum blending occurring typically between about 60 - about 80 rpm. Stirring occurs from about 60 minutes per addition, with a typical processing time of about 8 hours to about 24 hours per 10 litres/kg to 10,000 litre/kg blends the latter being dependent on using an appropriate mixing vessel. The first part of the process can be conducted at RT (about 20°C) for the organic acid addition steps, but is raised to about 50°C (45°C - 55°C) for flavonoid and polyol addition. Alternatively, the process can be started at about 50°C (45°C - 55°C) and maintained until all powders are fully solubilised. A maximum of 55°C, preferably 50°C permits full dissolution in the stated timeline and imposes no operational or structural damage to the flavonoids. Temperature selection was based on evidence of composition stability at max temp, rather than extensive variable analysis. Aeration and flocculation are minimised by application of a gentle mixing process (≤ 60 rpm, preferably ≤ 60-80 rpm)) with a limited range of blending blades. Excessive aeration and flocculation as elements of sub-optimal mixing can reduce solubility and enhance flavonoid oxidation, which is visible by absorbance and colour status. Exceeding the approved temperature will potentially reduce flavonoid function and concentration and enhance vapourisation with further concentration impact and reduction of biocidal efficacy. An example of an HPLC chromatogram of a completed product compliant with C-activ-12 characterisation is shown Figure 3.

### Preparation of C-Activ-NF biocide concentrate & Preferred E12/C-Activ-12 working solution

**(C-Activ-NF** (batch 25kg)) - Add water (7.750kg, 31% w/w) and put on stirrer (80rpm, 50°C). Add glycerine (5.375kg, 21.5% w/w 50°C) and stir for 15 minutes until flocculation disappears and solution is clear and allow system to re-attain 50°C for minimum 10 minutes. Add malic acid (4.380kg, 17.52% w/w, 50°C) and stir for 30-40 minutes at min 60-80rpm (or until the solution has become clear and solution has re-attained 50-55°C). Add citric acid (4.380kg, 17.52% w/w, 50°C) and stir for 30-40 minutes at min 60-80rpm (or until the solution has become clear and solution has re-attained 45-55°C). Add ascorbic acid (1.880kg, 7.52% w/w, 50°C) and stir for 30-40 minutes at min 60-80 rpm (or until the solution has become clear and solution has re-attained 50-55°C). Add the flavonoids extract (1.250kg, 5% w/w of mimimum 2.5% w/w flavonoids, 50°C) and stir for 30-40 minutes at min 60-80rpm (or until the solution has become clear and solution has re-attained 50-55°C).

**E12/C-Activ-12** (25kg) - Add 15kg, 60%w/w water to tank and stir. Add 2.5kg, 10% w/w citric acid and stir until liquid is clear (minimum 30 minutes). Add 2.5kg, 10% w/w C-Activ-NF and stir for 30 minutes on low speed (60-80rpm). Add a further 5kg, 20% w/w of water and stir for 15-20 mins on low speed (60-80rpm). Seal and Label appropriately. Checked with HPLC and LC-MS analysis to determine the bioflavonoid types present in the final formulations.

**Routine Analytical, Shelf Life and Stability Data for Selected Phase 1 Formulation (indicating process repeatability to give a stable consistent product and indicates repeatability of the process)**

| | | | | | | | | |
|---|---|---|---|---|---|---|---|---|
| Analytical data on the NF Batch NF09008_26FEB2010 | | | | | | | | |

| **Test Item** | | | **RESULT** | | | **Specification** | | **Comment** |
|---|---|---|---|---|---|---|---|---|
| pH | | | 1.4 | | | 1.0-2.0 | | CONFORMS |
| Flavonoids Content (UV) | | | 3.2% | | | > 2.5% | | CONFORMS |
| Acids Content | | | 41.3% | | | > 35% | | CONFORMS |
| Glycerine Content | | | 23.6% | | | < 25% | | CONFORMS |
| Water Content (LOD) | | | 34.3% | | | < 35% | | CONFORMS |
| Water Solubility | | | Complete | | | Complete | | CONFORMS |
| Appearance | | | Dark Brown Liquid | | | Dark Brown Liquid | | CONFORMS |
| Specific Gravity @ 20°C | | | 1.21 | | | 1.20-1.25 | | CONFORMS |
| Viscosity @ 20°C | | | 47 | | | 20-100cP | | CONFORMS |

| Analytical data on the C-Activ-12/E12 Batch E1209008_26FEB2010 | | | | | | | | |
|---|---|---|---|---|---|---|---|---|
| **Test Item** | | **RESULT** | | **Comment** | | | | |
| pH | | 1.8 | | CONFORMS | | | | |
| Flavonoids Content (UV) | | 3.2% | | CONFORMS | | | | |
| Acids Content | | 14.6% | | CONFORMS | | | | |
| Glycerine Content | | 2.5% | | CONFORMS | | | | |
| Water Content (LOD) | | 83.4% | | CONFORMS | | | | |
| Water Solubility | | Complete | | CONFORMS | | | | |
| Appearance | | Dark Brown Liquid | | CONFORMS | | | | |

| Stability Data - C-Activ-12 (E12) - AFTER 6 months | | | | | | | | |
|---|---|---|---|---|---|---|---|---|
| **Month** | **RESULT** | | | | **Comment** | | | |
| 1 | No visual change / No growth | | | | NO PPT | | | |
| 2 | No visual change / No growth | | | | NO PPT | | | |
| 3 | No visual change / No growth | | | | NO PPT | | | |
| 4 | No visual change / No growth | | | | NO PPT | | | |
| 5 | No visual change / No growth | | | | NO PPT | | | |
| 6 | No visual change / No growth | | | | NO PPT | | | |
| 7 | Small precipitation - back into solution after shaking / No growth | | | | PPT | | | |
| 8 | Small precipitation - back into solution after shaking / No growth | | | | PPT | | | |

| Stability Data - NF after 6 months | | | | | | | | |
|---|---|---|---|---|---|---|---|---|
| **Month** | | **RESULT** | | | | | **Comment** | |
| 1 | | No visual change / No growth | | | | | NO PPT | |
| 2 | | No visual change / No growth | | | | | NO PPT | |
| 3 | | No visual change / No growth | | | | | NO PPT | |
| 4 | | No visual change / No growth | | | | | NO PPT | |
| 5 | | No visual change / No growth | | | | | NO PPT | |
| 6 | | No visual change / No growth | | | | | NO PPT | |
| 7 | | No visual change / No growth | | | | | NO PPT | |
| 8 | | No visual change / No growth | | | | | NO PPT | |
| 9 | | No visual change / No growth | | | | | NO PPT | |

***Summary of Test Data using CeBeC Active 12 (C-Activ-12)*** -The C-Activ-NF concentrate composition has shown consistent efficacy against harmful micro-organisms, whilst the working solution C-Activ-12 formulation has shown consistent high log reductions of selected gram positive and negative bacterial strains, performing and meeting log reductions under BSEN1276 suspension test for chemical disinfectants and antiseptics. C-activ-12 also achieved the required log reductions under surface dried bacteria testing requirements, isolated bacteria and bacteria in bio-film, as supported under independent testing by University College Dublin Department of Food Safety & Zoonotic Diseases when the invention formulations were tested and compared against chemical based toxic products available on the market under a FIRM (Food Institutional Research Measure) Department of Food and Marine funded study. As a novel natural biocide, C-Activ-12 performed better than the chemically synthesized alternatives. The study also found that C-Activ-12 has a zero resistance profile, i.e. it does not cause mutations and has multiple modes of action, targeting a number of sites of the harmful micro-organsisms, with a low MIC and a total MBC with extended exposure time. The biocial compositions of the invention have been evaluated in four separate research institutions since 2009 (as per available test & laboratory reports) with a high level of correlative agreement regarding biophysical properties and gram negative/positive log reduction effective profile. The tests confirm high activity of the biocides of the invention against a range of both gram-positive and gram-negative bacteria. This highlights the exceptionally useful biocidal activity of the compositions arising from, the process of the invention.

### Summary of Initial Biocide Findings for Highly Active CeBeC Active 12 (C-Activ-12) Composition

Initial efforts were concentrated on two zoonotic pathogens namely verocytoxigenic *Escherichia coli* and *Salmonella* species, along with the recently described powdered infant formula (PIF) pathogen *Cronobacter* species. All isolates analysed (in each case > 100 were selected from strains recovered during various epidemiological studies) were found to be susceptible to biocides in common use in the meat and PIF industries. Some of the genera appeared to be more susceptible compared with others, as in the case of *Cronobacter.* Attempts to develop tolerant phenotypes were marginally successful and where this was generated, the phenotype was unstable. As a means of modelling the increased tolerance to biocide-active compounds, a small number of isolates were challenged with increasing concentrations of actives such as triclosan, chlorhexidine and benzalkonium chloride. Highly-tolerant mutant phenotypes were obtained and these are currently being studied. It was decided that it would be of interest to include the poultry food-borne zoonotic pathogen *Campylobacter* and to evaluate its susceptibility/tolerance to the same panel compounds. Once again, it could be shown that *Campylobacter* was susceptible. However, as the panel of biocidal compounds used could be described as synthetic, the availability of C-Activ-12 natural composition of the invention presented an opportunity to re-evaluate the responses of these bacteria to the natural biocide composition of the invention. Initially planktonically-growing *campylobacter* were tested and found to be completely susceptible to C-Activ-12 at working concentrations ranging from 0.5 through 1% (w/v). Similarly when these same cells were part of a biofilm, C-Activ-12 remained effective over the latter concentration range. These studies were subsequently extended to include *Cronobacter, Salmonella* and *E. coli* (as outlined above) and in each case the bacteria tested were found to be susceptible to the natural biocide product (C-Activ-12) at working concentrations, being bacteriocidal in all cases. C Activ-12 has also been subject to an *in vivo* poultry trial to assess its capacity to moderate indigenous *Campylobacter* population (Foundation of Research Science & Technology (New Zealand) - Study on Broiler Chickens at the University of Sydney, trial conducted by Dr Peter Groves Vice of the Poultry Vets Association). In a preliminary anti-Campylobacter live poultry trial, 800 one day old broiler chicks were exposed to low sub-approval dose (0.1%/ 1,000ppm) to test C-Activ-12 biocide in drinking water over a controlled 42 day trial. The addition of C Activ-12 to drinking water continuously for broiler chickens showed no deleterious effects the birds, on growth rate, feed efficiency or visceral organ weights, and was readily consumed by the birds. A major mediator of poultry *Campylobacter* transfer infection is exposure to contaminated faeces. In this pre-trial, there was a significant decline in the level of *C. jejuni* in the caecae (the gut of the bird) consistent with the level found in faeces, this was inversely associated with the dose rate of C Activ-12 after a simulated transport stress. Furthermore, propagation of *Campylobacter* infestation of poultry is influenced by faecal release (Ogden et al 2009). The biocide composition at working concentration in this project has shown a capacity at sub-MIC concentration in an independent animal trial to significantly reduce faecal bacterial density and also exhibited a high capacity to terminate a recently evaluated food pathogen in Dairy processing called *Cronobacter* species.

### Further Biocide Activity Studies

Six bacterial strains were tested according to BS EN 1276, at working concentration of ≤ 1% (w/v), namely *Salmonella typhimurium* ATCC 14028, *Enterococcus hirae* ATCC 8048, *Listeria monocytogenes* NCTC 11994, *Pseudomonas aeruginosa* ATCC 15442, *Escherichia coli* ATCC 25922 and *Stapylococcus aureus* ATCC 25923. The gram -ve *Escherichia coli, Salmonella typhimurium, Pseudomonas aeruginosa,* reproducibly showed a 5 log₁₀ reduction at the biocidal composition working concentration, while the gram +ve strains (all of which are clinically relevant) showed a 6 log₁₀ decline.

In a further study, a number of biocide compositions at 1% to 2% concentrations were tested against a set of five similar enteric pathogens, *Campylobacter jejuni* NCTC 11392 *C*. *sakazakii* ATCC 13076, *S*. *typhimurium* CCC2, *E. coli NCTC 1093, S. pollorum* ATCC 19945, using a well diffusion assay (Martín et al. (2009)). All strains demonstrated clear susceptibility to the biocide composition of the invention. In the case of the enteric pathogens/ surrogates inhibition zones >3mm were consistently recorded over 3 replicates when tested against the biocide (1:10 dilution corresponding to a working concentration of the biocide composition concentrate of the invention). The zones of clearing were tested for bacteriocidal/bacteristatic activity by stabbing the zone of clearing and streaking on to non-selective agar followed by selective agar. No growth was observed from the zones of clearing. While only one replicate was carried out for the dental pathogen, the biocide composition of the invention also proved effective against the majority of the dental pathogens. On average about 2 mm zones of clearing were observed when tested against the "working composition" (1:10 dilution). Again all zones of clearing tested suggested that the activity at this level was bacteriocidal at the 1/10 working concentration of biocide.

In an efficacy stability study measured against *E. coli,* the biocide composition- showed functional stability after been frozen and thawed (between 0 and 42°C for ~ 24 hrs) and after heating up and cooling.

In a separate study (Epidimiology study on Campylobacter Isolates in the Republic of Ireland, UCD; University of Sydney study testing the reductions of the biocidal composition of reducing the numbers of Campylobacter and Salmonella in Poultry), employing high throughput screening of natural isolates of and the other wildtypes samples isolated in an epidemiology study of poultry highlighted the efficacy of the natural biocide of the invention. No growth was uniquely recorded at 0.5 and 1% operative concentrations of the biocide. It was not confirmed, whether the latter was bacteriostatic or bacteriocidal. However, a study undertaken by a partner has provided dose evidence of significant bacteriocidal actions.

### Evaluation of Bactericidal Efficacy of the Biocide Compositions of the Invention

Tests have indicated that the compositions of the invention act as broad spectrum anti-bacterial agents effective against gram positive and gram negative bacteria (BSEN 1276). The compositions are suitable for use in the food and food processing industry. For rapid screening, a preliminary method allowed selection of the most efficient formulations. Two prototypes (E12 and G3 (E12 with a surfactant component)) were then tested using the standard method. The composition of each formulation (0.5% v/v) was used blind to ensure no bias. The formulation was reviewed after each set of tests to improve the products composition and efficiency. Prototype formulations were prepared under aseptic conditions.

| Culture conditions used for bacterial strains studied | | | | |
|---|---|---|---|---|
| ***Bacteria*** | ***Media*** | ***Incubation temperature (°C)*** | ***Incubation time (Hours)*** | ***Respiratory conditions*** |
| *Escherichia coli ATCC 25922 (gn)* | *Nutrient agar* | 37 | 24 | *Aerobic* |
| *Stapylococcus aureus ATCC 25923 (gp)* | *Nutrient agar* | 37 | 24 | *Aerobic* |
| *Salmonella typhimurium A TCC 14028 (gn)* | *Nutrient agar* | 37 | 24 | *Aerobic* |
| *Pseudomonas aeruginosa ATCC 15442 (gn)* | *Nutrient agar* | 37 | 24 | *Aerobic* |
| *Listeria monocytogenes NCTC 11994 (gp)* | *Columbia blood agar* | 37 | 24 | *Aerobic* |
| *Campylobacter jejuni NCTC* 11392 *(gn)* | *Blood agar base 2* | 42 | 72 | *Microaerophilic* |
| *Enterococcus hirae ATCC 8048(gp)* | *Columbia blood agar* | 37 | 48 | *Aerobic* |
| **Contact Time:** 5 mins ± 10 secs; **TestTemperature:** 20°C ± 0.5°C; **Product Storage:** 5°C in the dark | | | | |
| Reagents: Water: Free from toxins or bacterial inhibitors. Fresh distilled water, which had been sterilized by autoclaving. Hard-water (Product diluent): as per Abbott Analytical - 300 mg/kg CaC03 and sterilized by autoclaving. Neutralising Solution: 3% (w/v) Tween 80, 3% (w/v) Saponin, 0.1% (w/v) Histidine, 0.1% (w/v) Cysteine. Interfering Substance: (a) Clean conditions: 0.3 g BSA in 100 ml water, sterilized by membrane filtration (pore size 0.22 µm). Final concentration in the test procedure is 0.3 g/L. (b) Dirty conditions: 3 g BSA in 100 ml water, sterilized by membrane filtration. Final concentration in the test procedure is 3 g/L. | | | | |

| **Biocidal Efficacy** - A large number of formulations comprising *inter alia* flavonoids and food grade acids were prepared and tested. | | |
|---|---|---|
| *09008E1* | Flavonoid Mixture only | Single Strength |
| *09008E2* | Flavonoid Mixture ONLY | Double Strength |
| *09008E3* | MALIC ACID ONLY | Single Strength |
| *09008E4* | CITRIC ACID ONLY | Single Strength |
| *09008E5* | ASCORBIC ACID ONLY | Single Strength |
| ***09008E6*** | **Normal Formulation** | **Cold Blended** |
| *09008E7* | Normal Formulation | plus 10% EtOH |
| *09008E8* | Normal Formulation | 2 x Malic Acid |
| *09008E9* | Normal Formulation | 2 x Citric Acid |
| *09008E10* | Normal Formulation | 2 x Ascorbic Acid |
| *09008E11* | Normal Formulation | plus 20% EtOH |
| ***09008E12*** | **Normal Formulation** | **diluted to 10% + 10% Citric Acid** |
| *09008E13* | Normal Formulation | plus Double Strength Flavonoid Mixture |
| *09008E14* | Normal Formulation | plus 2% SDS |
| *09008E15* | Normal Formulation | 09008E14 plus Double Strength Flavonoid Mixture |

The most successful formulation was E12 formulation (C-activ-12). This was initially used neat as prepared from NF, as per other formulations, in concentrate form, but once it had shown effectiveness, the E12 dilutions were tested for Minimum Inhibitory Concentrations (MIC) under British & European testing standards. C-Activ-NF & C-Activ-12 were further diluted and were sent to for LC-MS analysis to compare both. The pre-blended bioflavonoid mixture starting material concentration was then doubled and the micro testing of this composition (E13) was repeated with no improvement in results. This confirms that the concentration in E12 provides the optimum activity. The addition of surfactant SDS (E14 - see results below) was expected to improve activity (surfactant component would be expected to enhance cell permeability, dispersion and associated adherence), but it was found not to improve the effectiveness of the materials. Ethanol was also added (E7 and E11) with no increase in effectiveness (see results below). The following re-formulations were also performed based on the most active E12 NF composition defined above. Note when ethanol and or SDS was added to E12, it maintained its effectiveness. This was an interesting finding as it highlights that the invention can be combined with adjuvants for varying applications - refer to Campylobacter epidemiology study described herein.

| | | Dilution |
|---|---|---|
| ***E12*** | Normal Formulation | 10% NF + 10% Citric Acid + 80% Water |
| ***E12+*** | Normal Formulation | 10% NF + 10% Citric Acid + 60°/ Water + 20% EtOH |
| ***E12S*** | Normal Formulation | 10% NF + 10% Citric Acid + 75% Water + 5% SDS |
| ***E12S+*** | Normal Formulation | 10% NF + 10% Citric Acid + 55% Water + 20% EtOH + 5% SDS |
| ***E12++*** | Normal Formulation | 10% NF + 10% Citric Acid + 40% Water + 40% EtOH |
| ***E12S++*** | Normal Formulation | 10% NF + 10% Citric Acid + 35% Water + 40% EtOH + 5% SDS |
| ***E12C+*** | Normal Formulation | 10% NF + 30% Citric Acid + 40% Water + 20% EtOH |

Each biocidal composition was tested blind. After each assay the formulation was adjusted as indicated above, and retested for inhibition of bacterial growth. A number of known sanitizers and detergent cleaners (labelled V1, W2, X3, Y4 and Z5 in these test) induced a log 5 reduction in microbiological tests, and these were assessed in preliminary tests for comparison purposes or as control.

### Preliminary Evaluation of Bactericidal Efficacy of CeBeC Natural Biocide Preliminary Formulations

Typically, an overnight culture of bacteria strains were diluted 1:1000 in sterile hard water to a final density of approx 1x10⁶ bacteria. A 1ml volume of bacteria was treated for 5 minutes at room temperature with 500 µl of antimicrobial solutions. The final volume was 1.5 ml so the final concentration of formulation e.g. E12 is 0.167 % (v/v). The treatment was ceased by the addition of 200 µl of the neutralising solution. All strains were serially diluted to 10⁻⁵ in sterile peptone water and 100µl were plated in duplicate on the appropriate plate and then incubated at 37°C for 24 hours except *Campylobacter jejuni* and *Enterococcus hirae.* The appropriate controls were included in each test e.g. an unopened agar plate and a set of serially diluted, untreated bacteria. After mesophilic aerobic incubation (or mesophilic microaerophilic incubation for *C. jejuni),* the number of colonies was enumerated and compared to the untreated controls to determine if a Log order reduction was obtained.

| Antimicrobial activity of selected commercial sanitisers as compared to solution E12 on E. coli | | | | | | | |
|---|---|---|---|---|---|---|---|
| | ***Escherichia coli*** | | | | | | |
| | **NEAT** | **10-1** | **10-2** | **10-3** | **10-4** | **10-5** | **10-6** |
| **Y4** | TNTC | TNTC | TNTC | 113 | 60 | 0 | 0 |
| **Z5** | TNTC | TNTC | TNTC | TNTC | 66 | 5 | 0 |
| **V1** | TNTC | TNTC | TNTC | TNTC | 0 | 0 | 0 |
| **X3** | TNTC | TNTC | TNTC | TNTC | TNTC | 1 | 0 |
| **E12** | TNTC | 0 | 0 | 0 | 0 | 0 | 0 |
| **Control** | TNTC | TNTC | TNTC | TNTC | TNTC | 0 | 0 |

**Conclusion:** It can be concluded that solution of the biocial composition of the invention E12 induced the greatest inhibition of bacterial growth of gram-negative E. coli in this test when compared to commercial sanitizers. The efficacy order of commercial solutions tested against *Escherichia coli* was: E12 > Y4 ≥ V1 ≥ Z5 > X3. E12 showed most activity with a reduction of 4 Log units.

| *E. coli* bacterial colony counts (average of two replicates) following 5 min exposure to 0.5% v/v of the various preliminary biocide formulations of the invention. | | | | | | |
|---|---|---|---|---|---|---|
| **Bacterial Dilution** | **Neat** | **10⁻¹** | **10⁻²** | **10⁻³** | **10⁻⁴** | **10⁻⁵** |
| **Antimicrobial Solution** | | | | | | |
| **Control** | TNTC | TNTC | TNTC | TNTC | TNTC | 1 |
| **E1** | TNTC | TNTC | TNTC | TNTC | TNTC | TNTC |
| **E2** | TNTC | TNTC | TNTC | TNTC | >14 | >5 |
| **E3** | TNTC | TNTC | TNTC | TNTC | TNTC | >61 |
| **E4** | TNTC | TNTC | TNTC | >159 | TNTC | 10 |
| **E5** | TNTC | TNTC | TNTC | TNTC | TNTC | TNTC |
| **E6** | TNTC | TNTC | TNTC | TNTC | TNTC | TNTC |
| **E7** | TNTC | TNTC | TNTC | TNTC | TNTC | 1 |
| **E8** | TNTC | TNTC | TNTC | TNTC | TNTC | >9 |
| **E9** | TNTC | TNTC | TNTC | TNTC | >113 | >114 |
| **E10** | TNTC | TNTC | TNTC | >82 | 2 | 2 |
| **E11** | TNTC | TNTC | TNTC | TNTC | TNTC | 2 |
| **E12** | 0 | 0 | 0 | 0 | 0 | 1 |
| **E13** | TNTC | TNTC | TNTC | TNTC | >5 | 3 |
| **E14** | TNTC | TNTC | TNTC | TNTC | TNTC | 0 |
| **E15** | TNTC | TNTC | TNTC | TNTC | TNTC | 53 |

**Conclusion:** It is evident from the results in above that the biocide formulation E12 has the most antimicrobial efficacy when treating gram negative *E. coli,* with an almost complete kill at all concentrations tested. In fact, E12 (C-Activ-12) produced a five log reduction of bacterial numbers in comparison with the control. E10 was responsible for a two log reduction, whereas E2, E9, E13 and maybe E8 produced just one log reduction. In view of the excellent results of E12 formulation in the preliminary tests, it was decided to test the E12 formulation, on a number of bacterial strains, at varying concentrations to further assess its efficacy.

| Bacterial colony counts (average of two replicates) following exposure to E12 formulations. | | | | | | |
|---|---|---|---|---|---|---|
| | ***Escherichia coli*** | | | | | |
| | **NEAT** | **10⁻¹** | **10⁻²** | **10⁻³** | **10⁻⁴** | **10⁻⁵** |
| **Control** | TNTC | TNTC | ≃ 450 | 69 | 14 | 2 |
| **E12** | 0 | 0 | 0 | 0 | 0 | 0 |
| **E12 (1/5)** | TNTC | 325 | 80 | 10 | 0 | 0 |
| **E12 (1/10)** | TNTC | TNTC | TNTC | ≃ 200 | 0 | 0 |

| | ***Salmonella typhimurium*** | | | | | |
|---|---|---|---|---|---|---|
| | **NEAT** | **10⁻¹** | **10⁻²** | **10⁻³** | **10⁻⁴** | **10⁻⁵** |
| **Control** | TNTC | TNTC | TNTC | 140 | 6 | 1 |
| **E12** | 1 | 0 | 0 | 0 | 0 | 0 |
| **E12 (1/5)** | 31 | TNTC | TNTC | ≃ 200 | 0 | 0 |
| **E12(1/10)** | TNTC | TNTC | TNTC | ≃ 282 | ≃ 119 | 0 |

| | ***Staphylococcus aureus*** | | | | | |
|---|---|---|---|---|---|---|
| | **NEAT** | **10⁻¹** | **10⁻²** | **10⁻³** | **10⁻⁴** | **10⁻⁵** |
| **Control** | TNTC | ≃ 500 | 122 | 15 | 1 | c |
| **E12** | 2 | 3 | 0 | 0 | 3 | 0 |
| **E12 (1/5)** | TNTC | ≃ 380 | 46 | 3 | 1 | 1 |
| **E12 (1/10)** | TNTC | ≃ 400 | 48 | 5 | 1 | 0 |

| | ***Pseudomonas aeruginosa*** | | | | | |
|---|---|---|---|---|---|---|
| | **NEAT** | **10⁻¹** | **10⁻²** | **10⁻³** | **10⁻⁴** | **10⁻⁵** |
| **Control** | TNTC | TNTC | 80 | 30 | 1 | 1 |
| **E12** | 0 | 0 | 0 | 0 | 0 | 0 |

| | ***Listeria monocytogenes*** | | | | | |
|---|---|---|---|---|---|---|
| | **NEAT** | **10⁻¹** | **10⁻²** | **10⁻³** | **10⁻⁴** | **10⁻⁵** |
| **Control** | TNTC c | TNTC | TNTC c | 115 | 18 | 1 |
| **E12** | 0 | 4 | 1c | 0 | 0 | 0c |

| | ***Enterococcus hirea*** | | | | | |
|---|---|---|---|---|---|---|
| | **NEAT** | **10⁻¹** | **10⁻²** | **10⁻³** | **10⁻⁴** | **10⁻⁵** |
| **Control** | TNTC | TNTC | TNTC | 91 | 17 | 0 |
| **E12** | 0 | 0 | 0 | 0 | 0 | 0 |

| | *Campylobacter jejuni* | | | | | |
|---|---|---|---|---|---|---|
| | **NEAT** | **10⁻¹** | **10⁻²** | **10⁻³** | **10⁻⁴** | **10⁻⁵** |
| **Control** | TNTC | TNTC | TNTC | 102 c | 13 | 4 |
| **E12** | 0 | 0 | 0 | 0 | 0 | 0 |
| C = plate with contamination | | | | | | |

**Conclusion:** From the results obtained it can be concluded that E12 is a very efficient formulation of the biocide compostion of the invention. E12 has the ability to inhibit the growth of all gram positive and gram negative bacterial strains tested when used as received (which is at a 0.5 % v/v). A second-generation batch of formulations, which were variations of E12 and were named 'F' was prepared using the E12 as a starting point.

| | | Variations |
|---|---|---|
| ***09008F1*** | 12g Water + 8g Glycerine | Bioflavonoid Mix (8g); Asc Acid (6g); Malic Acid (7g); Citric Acid (7g) |
| ***09008F2*** | 12g Water + 8g Glycerine | Pure Naringen (4g); Asc Acid (3g); Malic Acid (7g); Citric Acid (7g) |
| ***09008F3*** | 12g Water + 8g Glycerine | Bioflavonoid Mix (8g); Asc Acid (6g); Malic Acid (7g); Citric Acid (17g) |
| ***09008F4*** | 12g Water + 8g Glycerine | F3 + 100,000ppm SDS (2g/20mL Water) |
| ***09008F5*** | 12g Water + 8g Glycerine | F4 + 5% w/v Hypochlorite Bleach |
| ***09008F6*** | E12 (1 in 10 dilution) | Not Applicable |
| ***09008F7*** | E12 (1 in 5 dilution) | Not Applicable |
| ***09008F8*** | 12g Water + 8g Glycerine | F2 + Citric Acid @ 14g/10mL |
| ***09008F9*** | 12g Water + 8g Glycerine | F8 + 100,000ppm SDS |
| ***09008F10*** | 12g Water + 8g Glycerine | F9 + 5% w/v Hypochlorite Bleach |

These formulations were tested against the indicator species *S. typhimurium* and *E. coli* (see results below). The most effective solutions were tested on further organisms (see results below).

| ***Effects of F Compostion Variant against indicators species*** | | | | | | |
|---|---|---|---|---|---|---|
| | ***Salmonella typhimurium*** | | | | | |
| | **NEAT** | **10⁻¹** | **10⁻²** | **10⁻³** | **10⁻⁴** | **10⁻⁵** |
| Control | TNTC | TNTC | TNTC | ≃ 239 | > 22 | 2 |
| F1 | TNTC | TNTC | TNTC | > 177 | 40 | 16 |
| F2 | TNTC | TNTC | ≃ 450 | 67 | 12 | 0 |
| F3 | TNTC | TNTC | ≃ 400 | 80 | 4 | 1 |
| F4 | TNTC | 35 | 4 | 0 | 0 | 0 |
| F8 | TNTC | TNTC | ≃ 400 | 82 | 7 | 0 |
| F9 | TNTC | TNTC | 232 | 45 | > 10 | 0 |

| | *Escherichia coli* | | | | | |
|---|---|---|---|---|---|---|
| | NEAT | 10⁻¹ | 10⁻² | 10³ | 10⁻⁴ | 10⁻⁵ |
| Control | TNTC | TNTC | ≃ 450 | 69 | 14 | 2 |
| F1 | TNTC | TNTC | 328 | 54 | > 40 | 0 |
| F2 | TNTC | TNTC | > 157 | > 72 | 3 | > 3 |
| F3 | TNTC | TNTC | TNTC | > 61 | > 13 | 1 |
| F4 | 22 | 0 | 0 | 0 | 0 | 0 |
| F8 | TNTC | TNTC | 192 | 14 | 1 | 0 |
| F9 | TNTC | > 56 | 0 | 0 | 0 | 0 |

| | *Staphylococcus aureus* | | | | | |
|---|---|---|---|---|---|---|
| | NEAT | 10⁻¹ | 10⁻² | 10⁻³ | 10⁻⁴ | 10⁻⁵ |
| Control | TNTC | ≃ 500 | 122 | 15 | 1 | c |
| F4 | 7 | 0 | 0 | 0 | 0 | 0 |
| F9 | 3 | 0 | 0 | 0 | 0 | 0 |

| | *Pseudomonas aeruginosa* | | | | | |
|---|---|---|---|---|---|---|
| | NEAT | 10⁻¹ | 10⁻² | 10⁻³ | 10⁻⁴ | 10⁻⁵ |
| Control | TNTC | TNTC | 80 | 30 | 1 | 1 |
| F4 | 0 | 0 | 0 | 0 | 0 | 0 |
| F9 | 8 | 0 | 0 | 0 | 0 | 0 |

| | *Listeria monocytogenes* | | | | | |
|---|---|---|---|---|---|---|
| | NEAT | 10⁻¹ | 10⁻² | 10⁻³ | 10⁻⁴ | 10⁻⁵ |
| Control | TNTC c | TNTC | TNTC c | 115 | 18 | 1 |
| F4 | 75 | 6 | 0 | 0 | 1c | 0 |

| | *Enterococcus hirea* | | | | | |
|---|---|---|---|---|---|---|
| | NEAT | 10⁻¹ | 10⁻² | 10⁻³ | 10⁻⁴ | 10⁻⁵ |
| Control | TNTC | TNTC | TNTC | 91 | 17 | 0 |
| F4 | 95 | 15c 0 | | 0 | 0 | 0 |
| F9 | TNTC | TNTC | 121 | > 42 | 9 | 0 |
| | | | | | | |

| | *Campylobacter jejuni* | | | | | |
|---|---|---|---|---|---|---|
| | NEAT | 10⁻¹ | 10⁻² | 10⁻³ | 10⁻⁴ | 10⁻⁵ |
| Control | TNTC | TNTC | TNTC | 102c | 13 | 4 |
| F4 | 2 | 0c | 0 | 0c | 0 | 0c |

**Conclusion**: From the *E. coli* and *S. typhimurium* results it can be concluded that only the variations of E12, that is F4 and F9 show the best antibacterial activity. As a consequence, these 2 samples were tested on other bacterial strains that constitute the panel of organisms used in BSEN 1276. F4 produced a 5 Log reduction for *E. coli, P aeruginosa* and *C*. *jejuni,* a 4 Log reduction against *S. aureus* and *L. monocytogenes* and is responsible for a 3 Log reduction against *S. typhimurium* and *E. hirae.* F9 demonstrated more effective antibacterial activity against *P. aeruginosa* with 5 Log reduction of the bacterial population followed by *E. coli* and *S. aureus* (4 Log reductions). Unfortunately F9 was not efficient against *S. typhimurium* and *E. hirae.* Nevertheless, F4 and F9 samples contain Sodium Dodecyl Sulphate (SDS). As a consequence these solutions cannot be used to wash vegetables directly but could find application in sterilisation of worktops and equipments where food contact is indirect (SDS not suitable in food industry). In an effort to gain more information on E12, its main constituents were formulated into two third generation formulations designated G1 and G2 - effectively E12 with two SDS concentrations. In addition, another independent formulation was tested in this batch called G3.

| ***Antimicrobial effect of G formulations against indicator species.*** | | | | | | |
|---|---|---|---|---|---|---|
| | ***Escherichia coli*** | | | | | |
| | **NEAT** | **10⁻¹** | **10⁻²** | **10⁻³** | **10⁻⁴** | **10⁻⁵** |
| Control | TNTC | TNTC | TNTC | > 117 | 11 | 0 |
| G1 | 1 | TNTC | >96 | >120 | 0 | 0 |
| G2 | 9 | 68 | 22 | 1 | 0 | 0 |
| G3 | 42 | > 70 | 0 | 0 | 0 | 0 |

| | *Salmonella typhimurium* | | | | | |
|---|---|---|---|---|---|---|
| | NEAT | 10⁻¹ | 10⁻² | 10⁻³ | 10⁻⁴ | 10⁻⁵ |
| Control | TNTC | TNTC | TNTC | ≃ 280 | 54 | 0 |
| G1 | 4 | TNTC | 112 | 14 | 2 | 0 |
| G2 | 0 | 98 | 60 | 6 | 0 | 0 |
| G3 | 1 | 0 | 0 | 0 | 0 | 0 |

| | *Staphylococcus aureus* | | | | | |
|---|---|---|---|---|---|---|
| | NEAT | 10⁻¹ | 10⁻² | 10⁻³ | 10⁻⁴ | 10⁻⁵ |
| Control | TNTC | TNTC | 268 | 41 | 8 c | 1 |
| G 1 | ≃ 350 | 119 | 19 | 0 | 0 | 0 |
| G2 | TNTC | 333 | 40 | 14 | 1 | 0 |
| G3 | 0 | 0 | 0 | 0 | 0 | 0 |

| | *Pseudomonas aeruginosa* | | | | | |
|---|---|---|---|---|---|---|
| | NEAT | 10⁻¹ | 10⁻² | 10⁻³ | 10⁻⁴ | 10⁻⁵ |
| Control | TNTC | TNTC | 80 | 30 | 1 | 1 |
| G1 | 0 | 0 | 0 | 0 | 0 | 0 |
| G2 | 0 | 0 | 0 | 0 | 0 | 0 |
| G3 | 0 | 0 | 0 | 0 | 0 | 0 |

| | *Listeria monocytogenes* | | | | | |
|---|---|---|---|---|---|---|
| | NEAT | 10⁻¹ | 10⁻² | 10⁻³ | 10⁻⁴ | 10⁻⁵ |
| Control | TNTC c | TNTC | TNTC c | 115 | 18 | 1 |
| G3 | 0 c | 0 | 0 | 0 | 0 | 0 |

| | *Enterococcus hirea* | | | | | |
|---|---|---|---|---|---|---|
| | NEAT | 10⁻¹ | 10⁻² | 10⁻³ | 10⁻⁴ | 10⁻⁵ |
| Control | TNTC | TNTC | TNTC | 91 | 17 | 0 |
| G 1 | 97 | 9 | 0 | 0 | 0 | 0 |
| G2 | 5 | 0 | 0 | 0 | 0 | 0 |
| G3 | 0 | 26 | 0 | 0 | 0 | 0 |

| | *Campylobacter jejuni* | | | | | |
|---|---|---|---|---|---|---|
| | NEAT | 10⁻¹ | 10⁻² | 10⁻³ | 10⁻⁴ | 10⁻⁵ |
| Control | TNTC | TNTC | TNTC | 102 c | 13 | 4 |
| G3 | 0 | 0 | 0c | 0c | 0c | 0 |

**Conclusion:** G1 and G2 were shown to be less effective than their parent compound E12. G1 reduced bacterial population by 1 Log unit and G2 by 2 Log units against *E. coli* and *S*. *typhimurium* respectively. The E12 antibacterial effect is superior to that of G1 and G2 combined. This pattern was also observed against *E. hirae.* However, G1 and G2 were very efficient against *P. aeruginosa* giving a 6 Log order reduction. They may be recommended for *P. aeruginosa* specific applications and could lead to expanded product applications and lower cost in production.

### Test Report University Collge Dublin (UCD)

A sample of E12/C-Activ-12 formulated with other substances was tested on two strains of *C*. *Jejuni* in University College Dublin (UCD), using designated high throughput screening technology (HTS). 96 well plates were used to test a range of biocides against two strains of *Campylobacter jejuni* (strain NCTC 11168, other wildtype isolated from a poultry sample). A 1% v/v solution (in sterile distilled water) of the biocide was considered to be the working concentration. A range of the concentrations were prepared representing 200, 100, 50, 10, 1, 0.1 and 0.01 % of the 1% biocide working solution and added to a 96 well plate. Overnight bacterial suspentions diluted to give an inoculum of approx 10⁵ CFU per well were added to the 96 well plate. All strains were repeated in triplicate. The plate was incubated in microanaerobic coditions for 24 h at 37°C. A 96 pin replicator was then used to transfer a sample from the test wells to a 96 well plate filled with mCCDA. The mCCDA plate was incubated in microanaerobic coditions for 24 h at 37 °C and examined for growth. Results were recorded in wells where growth was observed. In all cases the biocidal activity of C-Activ-12 is maintained and is not inhibited. This is a significant qualifciation of the invention as it can be formulated with delivery technologies dependent on application. **Conclusions:** No cell growth was observed in any of the biocides when used at a 1% and 0.5 %. There was no difference observed between the ability of the type or wildtype strain in surviving biocide exposure.

**Conclusions: C-Activ-12** maintains its biocidal **activity.** No cell growth was observed in any of the biocides when used at a 1% and 0.5 %. There was no difference observed between the ability of the type or wildtype strain in surviving biocide exposure.

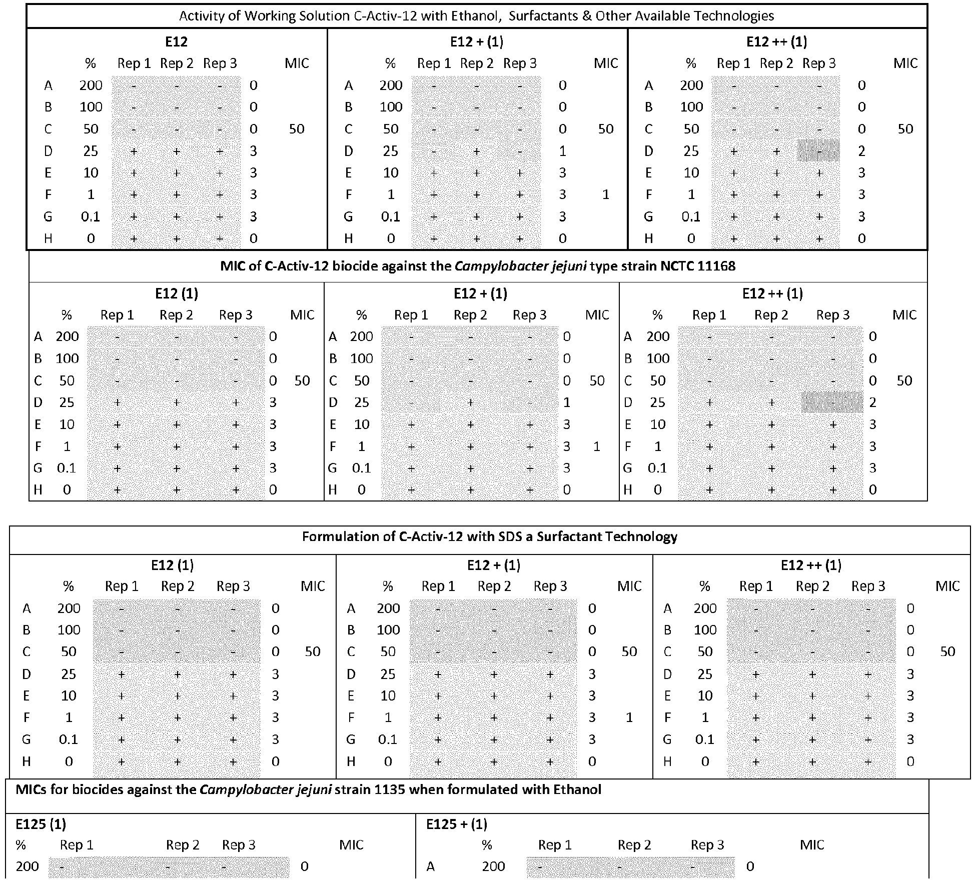

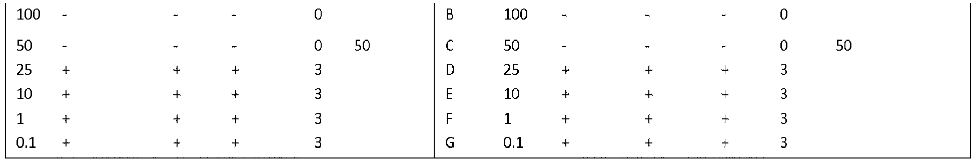

***Formulation factors*** -The active phytochemical concentration of NF is 5% (w/w) and C-activ-12 is 0.5% (w/w), and therefore a 1% working solution implies a phytochemical concentration of 0.005% (v/v) and a 0.5% solution implies a phytochemical concentration of 0.0025% (v/v). However, these values assume that the flavanol phytochemical content is 100% pure. For all antimicrobial test assays, a working solution was subject to further sequential dilutions and exposed to a fixed bacterial cell population in a given volume for a fixed and reproducible time. The exposed mixing stage results in a final operational dilution of the product - final concentration is 8 parts formulation to 2 parts interfering substance and bacterial population, implying a further one fifth dilution, e.g 0.5% (w/v) is at a final concentration of 0.4% (w/v) in the assay.

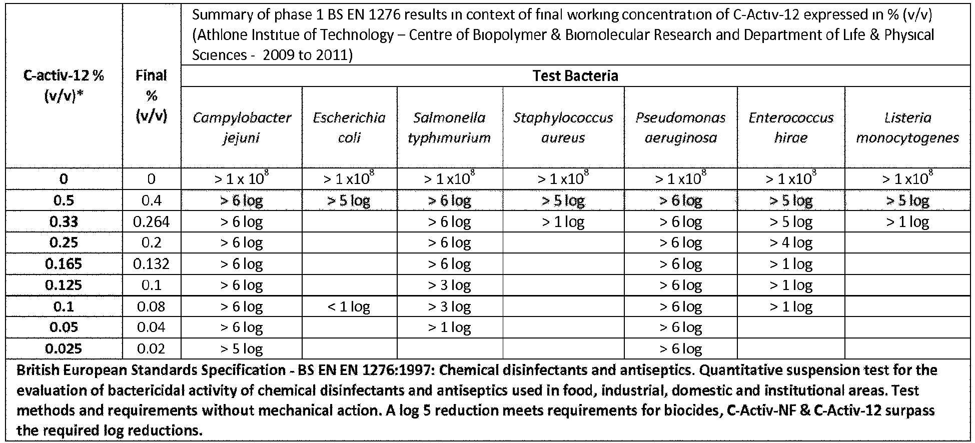

A comparison of comparative MIC values for different antimicrobial tests relative to common final working concentration of C-activ-12 expressed as a % dilution. Stock NF and C-activ-12 are prepared on a w/w basis, but subsequent dilutions are w/v and v/v, is shown below.

| **Summary of C-activ-12 MIC Values and Susceptibility (UCD FIRM Study 2009 - 2012** & **AIT Study 2009 - 2011)** | | | | | | |
|---|---|---|---|---|---|---|
| **Organism** | **Gram Status** | **Isolate identifier** | **MIC** | | | |
| | | | **BS EN 1276* (%)** | **High-through put screening * (%) Planktonic Surface-dried Biofilm** | | |
| | | | | | | |
| ***Campylobacter*** | | | | | | |
| | | | | | | |
| *\C. jejuni* | -ve | NCTC 11392 | 0.5 | | | |
| *C. jejuni* | -ve | NCTC 11168 | | 0.5 | 0.5 | 0.5 |
| *C. jejuni* | -ve | NCTC 1135 | | 0.5 | 0.5 | 0.5 |
| *C. jejuni* | -ve | Se 2 | | 0.5 | 0.5 | 0.5 |
| *C. jejuni* | -ve | Se 5 | | 0.5 | 0.5 | 0.5 |
| *C. jejuni* | -ve | Se 19 | | 0.5 | 0.5 | 0.5 |
| *C. jejuni* | -ve | Se 43 | | 0.5 | 0.5 | 0.5 |
| *C*. *coli* | -ve | Se 46 | | 0.5 | 0.5 | 0.5 |
| *C*. *coli* | -ve | Se 50 | | 0.5 | 0.5 | 0.5 |
| *C*. *coli* | -ve | Se 54 | | 0.0001 | 0.0001 | 0.0001 |
| *C. jejuni* | -ve | Se 77 | | 0.5 | 0.5 | 0.5 |
| *C. jejuni* | -ve | Se 83 | | 0.5 | 0.5 | 0.5 |
| *C. jejuni* | -ve | Se 84 | | 0.1 | 0.1 | 0.1 |
| *C. jejuni* | -ve | Se 85 | | 0.5 | 0.5 | 0.5 |
| *C. jejuni* | -ve | Se 86 | | 0.5 | 0.5 | 0.5 |
| *C. jejuni* | -ve | Se 96 | | 0.5 | 0.5 | 0.5 |
| *C*. *coli* | -ve | Se 185 | | 0.5 | 0.5 | 0.5 |
| *C.jejuni* | -ve | Se 226 | | 0.5 | 0.5 | 0.5 |
| *C*. *coli* | -ve | Se 241 | | 0.5 | 0.5 | 0.5 |
| *C*. *coli* | -ve | Se 246 | | 0.5 | 0.5 | 0.5 |
| *C*. *coli* | -ve | Se 266 | | 0.5 | 0.5 | 0.5 |
| *C*. *coli* | -ve | Se 279 | | 0.5 | 0.5 | 0.5 |
| *C*. *coli* | -ve | Se 311 | | 0.5 | 0.5 | 0.5 |
| *C*. *coli* | -ve | Se 316 | | 0.5 | 0.5 | 0.5 |
| *C*. *coli* | -ve | Se 330 | | 0.5 | 0.5 | 0.5 |
| *C. jejuni* | -ve | Se 341 | | 0.5 | 0.5 | 0.5 |
| *C. jejuni* | -ve | Se 366 | | 0.5 | 0.5 | 0.5 |
| *C. jejuni* | -ve | Se 376 | | 0.5 | 0.5 | 0.5 |

| *Salmonella* | | | | | | |
|---|---|---|---|---|---|---|
| *S. typhimurium* | -ve | ATCC 14028 | 0.165 | | | |
| Hadar | -ve | s6 | | 0.1 | 0.5 | 3.13 |
| Anatum | -ve | c28 | | 0.2 | 0.25 | 3.13 |
| Java | -ve | s43 | | 0.2 | 0.5 | 6.25 |
| Infantis | -ve | c9 | | 0.2 | 0.5 | 3.13 |
| *S. typhimurium* | -ve | s13 | | 0.2 | 0.25 | 3.13 |
| Dublin | -ve | s20 | | 0.2 | 0.25 | 6.25 |
| Anatum | -ve | c53 | | 0.2 | 0.5 | 3.13 |
| *S. typhimurium* | -ve | SL1344 | | 0.2 | 0.5 | 1.56 |

| *Cronobacter* | | | | | | |
|---|---|---|---|---|---|---|
| *C. sakazakii* | -ve | E543 | | 0.2 | 0.5 | 6.25 |
| *C. turicensis* | -ve | E866 | | 0.2 | 0.25 | 0.39 |
| *C. sakazakii* | -ve | E473 | | 0.2 | >0.5 | 3.13 |
| *C. sakazakii* | -ve | E491 | | 0.2 | 0.5 | 3.13 |
| *C. sakazakii* | -ve | Baa | | 0.2 | 0.5 | 3.13 |
| *C. dublinensis* | -ve | E515 | | 0.2 | 0.5 | 1.56 |
| *C. sakazakii* | -ve | E516 | | 0.2 | 0.5 | 3.13 |
| *C. turicensis* | -ve | E625 | | 0.2 | 0.5 | 6.25 |

| *Pseudomonas* | | | | | | |
|---|---|---|---|---|---|---|
| *P. aeruginoso* | -ve | ATCC 15442 | 0.025 | | | |
| *P. aeruginoso* | -ve | ATCC 15442 | | 1.56 | | |
| *P. aeruginoso* | -ve | ATCC 27853 | 2 | 0.78 | <0.78 | |

| ***Escherichia*** | | | | | | |
|---|---|---|---|---|---|---|
| *E. coli* | -ve | ATCC 10539 | 2.0 | 0.19 | | |
| *E. coli* | -ve | ATCC 25922 | 0.5 | | | |
| *E. coli* | -ve | ATCC 32518 | | 0.05 | 0.39 | |

| *Staphylococcus* | | | | | | |
|---|---|---|---|---|---|---|
| *S. aureus* | -ve | ATCC 25923 | 0.5 | | | |
| *S. aureus* | +ve | ATCC 6538 | 2 | 0.02 | | |
| *S. aureus* | +ve | ATCC 27853 | | 0.025 | 0.39 | |

| *Enterococcus* | | | | | | |
|---|---|---|---|---|---|---|
| *E. hirea* | +ve | ATCC 8048 | 0.33 | | | |
| *E. hirea* | +ve | ATCC 10541 | 2 | 1.56 | | |

| **Listeria** | | | | | | |
|---|---|---|---|---|---|---|
| *L. monocytogenes* | +ve | I NCTC 11994 | 0.5 | | | |
| Summary of mean MIC values and susceptibility testing technique for bacteria tested | | | | | | |

| **Organism** | **Gram Status** | **BS EN 1276*** | **MIC** | | | |
|---|---|---|---|---|---|---|
| | | | **High-through put screening *** | | | |
| | | | **Planktonic** | **Surface-dried** | **Biofilm** | |
| ***Campylobacter*** | | | | | | |
| *C. jejuni* | -ve | 0.5% | 0.5% | | 0.5% | |
| *C*. *coli* | -ve | | 0.5% | | | |

| ***Salmonella*** | | | | | | |
|---|---|---|---|---|---|---|
| Hadar | | | 0.1 | 0.5 | 3.13 | |
| Anatum | | | 0.2 | 0.25 | 3.13 | |
| Java | | | 0.2 | 0.5 | 6.25 | |
| Infantis | | | 0.2 | 0.5 | 3.13 | |
| Typhimurium | | | 0.2 | 0.25 | 3.13 | |
| Dublin | | | 0.2 | 0.25 | 6.25 | |
| Anatum | | | 0.2 | 0.5 | 3.13 | |
| Typhimurium | | | 0.2 | 0.5 | 1.56 | |

| ***Cronobacter*** | | | | | | |
|---|---|---|---|---|---|---|
| C. sakazakii | -ve | | 0.2 | 0.5 | 3.45 | |
| C. dublinensis | -ve | | 0.2 | 0.5 | 1.56 | |
| C. turicensis | -ve | | 0.2 | 0.5 | 4.69 | |

| ***Pseudomonas*** | | | | | | |
|---|---|---|---|---|---|---|
| *P. aeruginosa* | -ve | 2% | 1.17 | <0.78 | | |

| ***Escherichia*** | | | | | | |
|---|---|---|---|---|---|---|
| *E. coli* | -ve | 2% | 0.19% | 0.05 | 0.39 | |

| ***Staphylococcus*** | | | | | | |
|---|---|---|---|---|---|---|
| *S. aureus* | +ve | 2% | 0.022 | 0.39 | | |

| Enterococcus | | | | | | |
|---|---|---|---|---|---|---|
| *E. hirea* | +ve | 2 | 1.56 | | | |
| # cell density, 12 hr exposure, * not accredited; MIC- accepted ≥ 5 log reduction; Representative test data, of which Table?? is a summary is listed and concluded from p to p . | | | | | | |

| **Evaluation of Bactericidal Efficacy of Biocidal Compositions of the Invention Tested in Accordance with BSEN 1276 (Test Report Athlone Institute of Technology - May 2009)** | |
|---|---|
| **Bactericidal Activity Testing** | |
| **Sample:** | E12 Formulation (Brand name - C-Activ-12); |
| **Batch Number:** | E12May09 |
| **Manufacturer:** | CeBeC Group Ltd. |
| **Delivery Date:** | 8^{th} May 09 |
| **Product Storage:** | 5°C in the dark. |
| **Active Substance:** | Confidential |
| **Analysis Required:** | BSEN 1276 Dilution/Neutralization under dirty conditions. |
| | |

| **Experimental Conditions:** | |
|---|---|
| Product Diluent used during test: | Sterile hard water 300 mg/kg CaCO₃ |
| Product Test Concentration: | 5, 10, 20, 33.3, 50, 66.7 and 100 % (v/v) (MIC assessment) |
| Contact Time: | 5 min |
| Test Temperature: | 20°C ± 0.5°C |
| Interfering Substance: | 3.0 g/L Bovine albumin |
| Neutralising Solution: | 3% Tween 80, 3% Saponin, 0.1% Histidine, 0.1% Cysteine |
| | |

| **GRAM NEG** | |
|---|---|
| 1.Bacterial Strains: ***Campylobacter jejuni*** NCTC 11392; Temperature of Incubation: 42°C for 72 hours | |
| 2.Bacterial Strains: **Escherichia coli** ATCC 25922; Temperature of Incubation: 37°C for 24 hours | |
| 3.Bacterial Strains: **Salmonella typhimurium** ATCC 14028; Temperature of Incubation: 37°C for 24 hours | |
| 4.Bacterial Strains: **Pseudomonas aeruginosa** ATCC 15442; Temperature of Incubation: 37°C for 24 hours | |
| | |

| **GRAM POS** | |
|---|---|
| 5.Bacterial Strains: **Staphylococcus aureus** ATCC 25923; Temperature of Incubation:37°C for 24 hours | |
| 6.Bacterial Strains: **Enterococcus hirae** ATCC 8048; Temperature of Incubation:37°C for 24 hours | |
| **7.Bacterial** Strains: **Listeria monocytogenes;** Temperature of Incubation:37°C for 24 hours | |
| **NOTE:** BSEN 1276 requires 1ml of the neutralised test mixture to be overlaid with 12-15 ml of the appropriate molten agar. However, because Blood Agar Base 2 contains sterile defibrinated horse blood it is impossible to keep it molten, as the blood proteins coagulate in the oven and fall out of solution. Hence, the plates were pre-poured & the sample volume was adjusted to 100 µl as per standard spread plate method. | |
| **NOTE:** Cells shaded yellow indicate the lowest concentration which induces a ∼ 6 log order reduction (or greater) in bacterial viability. | |
| **NOTE: E12 is commercially referred to as CeBeC "C-Activ-12" Natural Sanitiser** | |
| **NOTE: 100% (v/v) represents 0.5% base dilution** | |

**Conclusions** - 1. According to BSEN 1276, E12 is extremely effective in reducing the viability of this strain of *Campylobacter jejuni* under dirty conditions. Almost a total kill was evident at all E12 dilutions down to 10% v/v solution or 0.1% MIC. However, a 5 log order reduction was still observed at the 5% v/v level or 0.05% MIC, indicating that the minimum inhibitory concentration (MIC) for E12 on *C*. *jejuni* is at 0.5 % v/v or slightly lower. 2.According to BSEN 1276, E12 is effective in reducing the viability of this strain of *Escherichia coli* under dirty conditions. There was a greater than 6 log reduction in bacterial viability at a concentration at 100 % test concentration of E12 as provided blindly to lab technician at a 2% V/V dilution of C-Activ-12/E12. There was a significant reduction in bacterial viability counts at the more dilute concentrations. **3.** According to BSEN 1276, E12 is effective in reducing the viability of this strain of *Salmonella typhimurium* under dirty conditions. There was a greater than 6 log reduction in bacterial viability at a concentration of 33.33 % v/v of the E12 test concentration as provided to the lab technician at a blind working concentration of 2%. There was a slight reduction in bacterial viability counts at the more dilute concentrations. **4.** According to BSEN 1276, E12 is extremely effective in reducing the viability of this strain of *Pseudomonas aeruginosa* under dirty conditions. A reduction of greater than 6 log order was observed down to a 5 % v/v dilution of the E12 solution. Therefore, the MIC for this strain of bacteria is ≤ 5 % v/v of the test concentration provided. There was a greater than 6 log reduction in bacterial viability at a concentration of 5 % v/v of the E12 test concentration as provided to the lab technician at a blind working concentration of 2%. 5.

According to BSEN 1276, E12 is effective in reducing the viability of this strain of *Staphylococcus aureus* under dirty conditions. There was a greater than 5 log reduction in bacterial viability at a concentration of 100 % v/v of the E12 test concentration as provided to the lab technician at a blind working concentration of 2%. There was a slight reduction in bacterial viability counts at the more dilute concentrations. 6. According to BSEN 1276, E12 is extremely effective in reducing the viability of this strain of *Enterococcus hirae* under dirty conditions. A reduction of 5 order log reduction was observed in bacterial viability at a concentration of 66.67 % v/v of the E12 test concentration as provided to the lab technician at a blind working concentration of 2% v/v E12 solution, making 1% the MIC value for this strain of bacteria. There was a slight reduction in viability counts at the more dilute concentration. 7. According to BSEN 1276, E12, at 100 % (v/v) is effective in reducing the viability of this strain of *Listeria monocytogenes* under dirty conditions. The results show an almost complete kill with the Neat solution. However, further dilutions of the E12 solution showed a slightly reduced viability, but not the required 5 log reduction

| **Test Results, BSEN 1276, *C. Jejuni ― Athlone Institue of Technology May 2009*** | | | | | | | | |
|---|---|---|---|---|---|---|---|---|
| **Bacterial Dilution** | **Neat** | **10⁻¹** | **10⁻²** | **10⁻³** | **10⁻⁴** | **10⁻⁵** | **10⁻⁶** | **10⁻⁷** |
| **Untreated control** | >3x10³ | >3x10³ | >3x10³ | V_{c} >10 Nₐ<1.5x10² | V_{c} 3 Nₐ<1.5x10² | V_{c} 0 Nₐ<1.5x10² | V_{c} 0 Nₐ<1.5x10² | V_{c} 0 Nₐ<1.5x10² |
| **E12 Conc. % (v/v)** | | | | | | | | |
| **100** | V_{c} 0 | V_{c} 0 | V_{c} 0 | V_{c} 0 | V_{c} 0 | V_{c} 0 | V_{c} 0 | V_{c} 0 |
| | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² |
| **66.7** | V_{c} 0 | V_{c} 0 | V_{c} 0 | V_{c} 0 | V_{c} 2 | V_{c} 0 | V_{c} 0 | V_{c} 0 |
| | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² |
| **50** | V_{c} 0 | V_{c} 0 | V_{c} 0 | V_{c} 0 | V_{c} 0 | V_{c} 0 | V_{c} 0 | V_{c} 0 |
| | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² |
| **33** | V_{c} 0 | V_{c} 0 | V_{c} 0 | V_{c} >1 | V_{c} 0 | V_{c} 0 | V_{c} 0 | V_{c} 0 |
| | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² |
| **25** | V_{c} 0 | V_{c} 0 | V_{c} 0 | V_{c} 0 | V_{c} 0 | V_{c} 0 | V_{c} 0 | V_{c} 0 |
| | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² |
| **20** | V_{c} 0 | V_{c} 0 | V_{c} 0 | V_{c} 0 | V_{c} 0 | V_{c} 0 | V_{c} 0 | V_{c} 0 |
| | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² |
| **10** | V_{c} 0 | V_{c} 0 | V_{c} 0 | V_{c} 0 | V_{c} 0 | V_{c} 0 | V_{c} 0 | V_{c} 0 |
| | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² |
| **5** | | | V_{c} 3 | V_{c} 0 | V_{c} 1 | V_{c} 0 | V_{c} 0 | V_{c} 0 |
| | 2.29 x10³ | 2.0 x 10² | Nₐ<1.5x10² | Nₐ<1.5x10² | Nₐ<1.5x10² | Nₐ<1.5x10² | Nₐ<1.5x10² | Nₐ<1.5x10² |
| *V*c = viable count; *N*a = number of cfu/ml in the test mixture **NOTE**: The BSEN 1276 standard dictates that colony counts of less than 15 are represented by < 1.5 x 10² and that counts of over 300 be presented as > 3 x 10³ | | | | | | | | |

| **Summary threshold results, *C. jejuni*** | | | **Test Results BSEN 1276, *E*.** *Coli* | | | **Summary threshold results, *S*. *typhimurium*** | | |
|---|---|---|---|---|---|---|---|---|
| **E12 Blind Test** | | **Reduction in Viability** | **E12 Concentration %** | **Reduction in Viability** | | **E12 Concentration** | | **Reduction in** |
| **0** | | > 1 x 10⁸ | **0** | > 1 x 10⁸ | | **0** | | > 1 x 10⁸ |
| **100%** | | > 6 log | **100** | > 6 log | | **100** | | > 6 log |
| **66.67** | | > 6 log | **66.67** | ≥ 3 log | | **66.67** | | > 6 log |
| **50** | | > 6 log | **50** | > 2 log | | **50** | | > 6 log |
| **33.33** | | > 6 log | **33.33** | > 2 log | | **33.33** | | > 6 log |
| **25** | | > 6 log | **25** | < 1 log | | **25** | | > 3 log |
| **20** | | > 6 log | 20 | < 1 log | | **20** | | > 3 log |
| **10** | | > 6 log | 10 | < 1 log | | 10 | | > 1 log |
| **5** | | > 5 log | 5 | < 1 log | | 5 | | - |
| **Summary threshold results, *E. coli*** | | | | | | | | |
| **Bacterial Dilution** | **Neat** | **10⁻¹** | **10⁻²** | **10⁻³** | **10⁻⁴** | **10⁻⁵** | **10⁻⁶** | **10⁻⁷** |

| **E12 Conc. % (v/v)** | | | | | | | | |
|---|---|---|---|---|---|---|---|---|
| **100** | V_{c} 0 | V_{c} 5 | V_{c} 0 | V_{c} 0 | V_{c} 0 | V_{c} 0 | V_{c} 0 | V_{c} 0 |
| | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² |
| **50** | V_{c} >300 | V_{c} >300 | V_{c} >300 | V_{c} 131 | V_{c} 4 | V_{c} 0 | V_{c} 0 | V_{c} 0 |
| | Nₐ>3x 10³ | Nₐ>3x 10³ | Nₐ>3x 10³ | Nₐ1.31x10⁵ | Nₐ<1.5x10² | Nₐ < 1.5 x10² | Nₐ < 1.5 x10² | Nₐ < 1.5 x10² |
| **33** | V_{c} >300 | V_{c} >300 | V_{c} >300 | V_{c} >300 | V_{c} 40 | V_{c} 0 | V_{c} 0 | V_{c} 0 |
| | Nₐ>3x 10³ | Nₐ>3x 10³ | Nₐ>3x 10³ | Nₐ>3x 10³ | Nₐ 4.0x10⁵ | Nₐ < 1.5 x10² | Nₐ < 1.5 x10² | Nₐ < 1.5 x10² |
| **25** | V_{c} >300 | V_{c} >300 | V_{c} >300 | V_{c} >300 | V_{c} >300 | V_{c} 52 | V_{c} 1 | V_{c} 0 |
| | Nₐ>3x 10³ | Nₐ>3x 10³ | Nₐ>3x 10³ | Nₐ>3x 10³ | Nₐ>3x 10³ | Nₐ 5.2 x10⁶ | Nₐ < 1.5 x10² | Nₐ < 1.5 x10² |
| **20** | V_{c} >300 | V_{c} >300 | V_{c} >300 | V_{c} >300 | V_{c} 46 | V_{c} 7 | V_{c} 1 | V_{c} 0 |
| | Nₐ>3x 10³ | Nₐ>3x 10³ | Nₐ>3x 10³ | Nₐ>3x 10³ | Nₐ 4.6x 10⁵ | Nₐ < 1.5 x10² | Nₐ < 1.5 x10² | Nₐ < 1.5 x10² |
| **10** | V_{c} >300 | V_{c} >300 | V_{c} >300 | V_{c} >300 | V_{c} >300 | V_{c} 65 | V_{c} 4 | V_{c} 0 |
| | Nₐ>3x 10³ | Nₐ>3x 10³ | Nₐ>3x 10³ | Nₐ>3 x 10³ | Nₐ>3x 10³ | Nₐ 6.5 x10⁶ | Nₐ < 1.5 x10² | Nₐ < 1.5 x10² |
| **5** | V_{c} >300 | V_{c} >300 | V_{c} >300 | V_{c} >300 | V_{c} >300 | V_{c} 68 | V_{c} 4 | V_{c} 0 |
| | Na>3x10³ | Nₐ>3x10³ | Nₐ>3x10³ | Nₐ>3x10³ | Nₐ>3x10³ | Nₐ 6.8 x10⁶ | Nₐ < 1.5 x10² | Nₐ<1.5x10² |
| *V*c = viable count *N*a = number of cfu/ml in the test mixture **NOTE:** The BSEN 1276 standard dictates that colony counts of less than 15 are represented by < 1.5 x 10² and that counts of over 300 be presented as > 3 x 10³. | | | | | | | | |
| **Test Results BSEN 1276 *S*. *Typhimurium*** | | | | | | | | |
| **Bacterial Dilution** | **Neat** | **10⁻¹** | **10⁻²** | **10⁻³** | **10⁻⁴** | **10⁻⁵** | **10⁻⁶** | **10⁻⁷** |
| **Untreated** | V_{c} > 300 | V_{c} > 300 | V_{c} >300 | V_{c} >300 | V_{c} >300 | V_{c} 30 | V_{c} 5 | V_{c} 3 |
| **control** | Nₐ>3x10³ | Nₐ>3x10³ | Nₐ>3x10³ | Nₐ>3x10³ | Nₐ>3x10³ | Nₐ 3.0x10⁶ | Nₐ<1.5x10² | Nₐ<1.5x10² |

| **E12 Conc. % (v/v)** | | | | | | | | |
|---|---|---|---|---|---|---|---|---|
| **100** | V_{c} 0 | V_{c} 0 | V_{c} 0 | V_{c} 0 | V_{c} 0 | V_{c} 0 | V_{c} 0 | V_{c} 0 |
| | Nₐ<1.5x10² | Nₐ<1.5x10² | Nₐ<1.5x10² | Nₐ<1.5x10² | Nₐ<1.5x10² | Nₐ<1.5x10² | Nₐ < 1.5 x10² | Nₐ< 1.5 x10² |
| **66.7** | V_{c} 0 | V_{c} 2 | V_{c} 1 | V_{c} 0 | V_{c} 0 | V_{c} 0 | V_{c} 0 | V_{c} 0 |
| | Nₐ<1.5x10² | Nₐ< 1.5x10² | Nₐ<1.5x10² | Nₐ<1.5x10² | Nₐ<1.5x10² | Nₐ< 1.5 x10² | Nₐ< 1.5 x10² | Nₐ< 1.5 x10² |
| **50** | V_{c} 0 | V_{c} 0 | V_{c} 0 | V_{c} 0 | V_{c} 0 | V_{c} 1 | V_{c} 0 | V_{c} 0 |
| | Nₐ< 1.5 x10² | Nₐ< 1.5 x10² | Nₐ<1.5x10² | Nₐ<1.5x10² | Nₐ<1.5x10² | Nₐ< 1.5 x10² | Nₐ< 1.5 x10² | Nₐ < 1.5 x10² |
| **33** | V_{c} 1 | V_{c} 0 | V_{c} 0 | V_{c} 0 | V_{c} 0 | V_{c} 0 | V_{c} 0 | V_{c} 0 |
| | Nₐ< 1.5 x10² | Nₐ< 1.5 x10² | Nₐ<1.5x10² | Nₐ<1.5x10² | Nₐ<1.5x10² | Nₐ< 1.5 x10² | Nₐ< 1.5 x10² | Nₐ< 1.5 x10² |
| **25** | V_{c} 17 | V_{c} 118 | V_{c} 51 | V_{c} 3 | V_{c} 0 | V_{c} 0 | V_{c} 0 | V_{c} 0 |
| | Nₐ1.7x10¹ | Nₐ1.18 x 10³ | Nₐ5.1x 10³ | Nₐ<1.5x10² | Nₐ<1.5x10² | Nₐ< 1.5 x10² | Nₐ< 1.5 x10² | Nₐ< 1.5 x10² |
| **20** | V_{c} >300 | V_{c} >300 | V_{c} 67 | V_{c} 7 | V_{c} 0 | V_{c} 1 | V_{c} 0 | V_{c} 0 |
| | Nₐ>3x10³ | Nₐ>3x10³ | Nₐ6.7x10³ | Nₐ<1.5x10² | Nₐ<1.5x10² | Nₐ< 1.5 x10² | Nₐ< 1.5 x10² | Nₐ< 1.5 x10² |
| **10** | V_{c} >300 | V_{c} >300 | V_{c} >300 | V_{c} >300 | V_{c} 199 | V_{c} 21 | V_{c} 1 | V_{c} 0 |
| | Nₐ>3x10³ | Nₐ>3x10³ | Nₐ>3x10³ | Nₐ>3x 10³ | Nₐ1.99x10⁶ | Nₐ2.1 x10⁶ | Nₐ< 1.5 x10² | Nₐ< 1.5 x10² |
| *V*c = viable count *N*a = number of cfu/ml in the test mixture **NOTE**: The BSEN 1276 standard dictates that colony counts of less than 15 are represented by < 1.5 x 10² and that counts of over 300 be presented as > 3 x 10³. | | | | | | | | |

| **Test Results BSEN 1276, *P. Aeruginosa*** | | | | | | | | |
|---|---|---|---|---|---|---|---|---|
| **Bacterial Dilution** | **Neat** | **10⁻¹** | **10⁻²** | **10⁻³** | **10⁻⁴** | **10⁻⁵** | **10⁻⁶** | **10⁻⁷** |
| **Untreated control** | V_{c} >300 Nₐ>3x 10³ | V_{c} >300 Nₐ>3x 10³ | V_{c} >300 Nₐ>3x 10³ | V_{c} >300 Nₐ>3x10³ | V_{c} >300 Nₐ>3x10³ | V_{c} >221 Nₐ 2.21 x 10⁶ | V_{c} 24 Nₐ 2.4 x10² | V_{c} 3 Nₐ< 1.5 x10² |
| **E12 Concentration % (v/v)** | | | | | | | | |
| **100** | V_{c} 0 Nₐ<1.5x10² | V_{c} 0 Nₐ<1.5x10² | V_{c} 0 Nₐ<1.5x10² | V_{c} 0 Nₐ< 1.5 x10² | V_{c} 0 Nₐ< 1.5 x10² | V_{c} 0 Nₐ< 1.5 x10² | V_{c} 0 Nₐ< 1.5 x10² | V_{c} 0 Nₐ< 1.5 x10² |
| **66.7** | V_{c} 0 | V_{c} 0 | V_{c} 0 | V_{c} 0 | V_{c} 0 | V_{c} 0 | V_{c} 0 | V_{c} 0 |
| | Nₐ<1.5x10² | Nₐ<1.5x10² | Nₐ<1.5x10² | Nₐ< 1.5 x10² | Nₐ< 1.5 x10² | Nₐ< 1.5 x10² | Nₐ< 1.5 x10² | Nₐ< 1.5 x10² |
| **50** | V_{c} 0 | V_{c} 2 | V_{c} 0 | V_{c} 0 | V_{c} 0 | V_{c} 0 | V_{c} 0 | V_{c} 0 |
| | Nₐ<1.5x10² | Nₐ<1.5x10² | Nₐ<1.5x10² | Nₐ< 1.5 x10² | Nₐ< 1.5 x10² | Nₐ< 1.5 x10² | Nₐ< 1.5 x10² | Nₐ< 1.5 x10² |
| **33** | V_{c} 2 | V_{c} 1 | V_{c} 0 | V_{c} 0 | V_{c} 0 | V_{c} 0 | V_{c} 0 | V_{c} 0 |
| | Nₐ<1.5x10² | Nₐ<1.5x10² | Nₐ<1.5x10² | Nₐ<1.5x10² | Nₐ< 1.5 x10² | Nₐ< 1.5 x10² | Nₐ< 1.5 x10² | Nₐ< 1.5 x10² |
| **25** | V_{c} 0 | V_{c} 0 | V_{c} 0 | V_{c} 1 | V_{c} 0 | V_{c} 0 | V_{c} 1 | V_{c} 0 |
| | Nₐ<1.5x10² | Nₐ<1.5x10² | Nₐ<1.5x10² | Nₐ<1.5x10² | Nₐ< 1.5 x10² | Nₐ< 1.5 x10² | Nₐ< 1.5 x10² | Nₐ< 1.5 x10² |
| **20** | V_{c} 0 | V_{c} 0 | V_{c} 0 | V_{c} 0 | V_{c} 0 | V_{c} 0 | V_{c} 0 | V_{c} 0 |
| | Nₐ<1.5x10² | Nₐ<1.5x10² | Nₐ<1.5x10² | Nₐ<1.5x10² | Nₐ< 1.5 x10² | Nₐ< 1.5 x10² | Nₐ< 1.5 x10² | Nₐ< 1.5 x10² |
| **10** | V_{c} 0 | V_{c} 0 | V_{c} 0 | V_{c} 0 | V_{c} 0 | V_{c} 0 | V_{c} 0 | V_{c} 0 |
| | Nₐ<1.5x10² | Nₐ<1.5x10² | Nₐ<1.5x10² | Nₐ<1.5x10² | Nₐ<1.5x10² | Nₐ< 1.5 x10² | Nₐ< 1.5 x10² | Nₐ< 1.5 x10² |
| **5** | V_{c} 6 | V_{c} 5 | V_{c} 0 | V_{c} 0 | V_{c} 0 | V_{c} 0 | V_{c} 0 | V_{c} 0 |
| | Nₐ<1.5x10² | Nₐ<1.5x10² | Nₐ<1.5x10² | Nₐ<1.5x10² | Nₐ<1.5x10² | Nₐ< 1.5 x10² | Nₐ< 1.5 x10² | Nₐ< 1.5 x10² |
| Vc = viable count *N*a = number of cfu/ml in the test mixture NOTE: The BSEN 1276 standard dictates that colony counts of less than 15 are represented by < 1.5 x 10² and that counts of over 300 be presented as > 3 x 10³. | | | | | | | | |

| **Test Results BSEN 1276, *S. aureus*** | | | | | | | | |
|---|---|---|---|---|---|---|---|---|
| **Bacterial Dilution** | **Neat** | **10⁻¹** | **10⁻²** | **10⁻³** | **10**⁻⁴ | **10⁻⁵** | **10⁻⁶** | **10⁻⁷** |
| **Untreated control** | V_{c} > 300 | V_{c} > 300 | V_{c} >300 | V_{c} >300 | V_{c} 174 | V_{c} 18 | V_{c} 2 | V_{c} 0 |
| | Nₐ>3x10³ | Nₐ>3x10³ | Nₐ>3x10³ | Nₐ>3x 10³ | Nₐ1.74x10⁶ | Nₐ1.8x10⁶ | Nₐ< 1.5 x10² | Nₐ< 1.5 x10² |

| **E12 Concentrati on % (v/v)** | | | | | | | | |
|---|---|---|---|---|---|---|---|---|
| **100** | V_{c} 0 | V_{c} 10 | V_{c} 5 | V_{c} 0 | V_{c} 0 | V_{c} 0 | V_{c} 0 | V_{c} 0 |
| | Nₐ< 1.5 x10² | Nₐ<1.5x10² | Nₐ<1.5x10² | Nₐ<1.5x10² | Nₐ<1.5x10² Nₐ | < 1.5 x10² | Nₐ< 1.5 x10² | Nₐ< 1.5 x10² |
| **66.7** | V_{c} >300 | V_{c} >300 | V_{c} >300 | V_{c} >300 | V_{c} >300 | V_{c} 114 | V_{c} 47 | V_{c} 4 |
| | Na>3x 10³ | Nₐ>3x 10³ | Nₐ>3x 10³ | Nₐ>3x 10³ | Nₐ>3x 10³ Nₐ | 1.14 x10⁷ | Nₐ4.7x10⁷ | Nₐ< 1.5 x10² |
| **50** | V_{c} >300 | V_{c} >300 | V_{c} 280 | V_{c} 32 | V_{c} 4 | V_{c} 1 | V_{c} 1 | V_{c} 0 |
| | Nₐ>3x 10³ | Nₐ>3x 10³ | Nₐ2.8x10⁴ | Nₐ3.2x10⁴ | Nₐ<1.5x10² Nₐ | <1.5 x10² | Nₐ< 1.5 x10² | Nₐ< 1.5 x10² |
| **33** | V_{c} >300 | V_{c} >300 | V_{c} >300 | V_{c} 277 | V_{c} 86 | V_{c} 19 | V_{c} 12 | V_{c} 3 |
| | Nₐ>3x 10³ | Nₐ>3x 10³ | Nₐ>3x 10³ | Nₐ 2.77x10⁵ | Nₐ8.6x10⁵ | Nₐ1.9x₁₀⁶ | Nₐ< 1.5 x10² | Nₐ< 1.5 x10² |
| **25** | V_{c} >300 | V_{c} >300 | V_{c} >300 | V_{c} >300 | V_{c} 39 | V_{c} 12 | V_{c} 5 | V_{c} 0 |
| | Na>3x 10³ | Nₐ>3x 10³ | Nₐ>3x 10³ | Nₐ>3x 10³ | Nₐ3.93x10⁵ Nₐ | < 1.5 x10² | Nₐ< 1.5 x10² | Nₐ< 1.5 x10² |
| **20** | V_{c} >300 | V_{c} >300 | V_{c} >300 | V_{c} 137 | V_{c} 23 | V_{c} 6 | V_{c} 4 | V_{c} 1 |
| | Nₐ>3x 10³ | Nₐ> 3 x 10³ | Nₐ>3x 10³ | Nₐ1.37x10⁵ | Nₐ2.3x 10⁵ Nₐ | < 1.5 x10² | Nₐ< 1.5 x10² | Nₐ< 1.5 x10² |
| **10** | V_{c} >300 | V_{c} >300 | V_{c} >300 | V_{c} >300 | V_{c} >300 | V_{c} 146 | V_{c} 87 | V_{c} 12 |
| | Nₐ>3x10³ | Nₐ>3x10³ | Nₐ>3x10³ | Nₐ>3x 10³ | Nₐ>3x10³ Nₐ | 1.46 x10⁷ | Nₐ8.7x10⁷ | Nₐ< 1.5 x10² |
| **5** | Vac>300 | V_{c} >300 | V_{c} >300 | V_{c} >300 | V_{c} >300 | V_{c} 70 | V_{c} 21 | V_{c} 2 |
| | Nₐ>3x10³ | Nₐ>3x10³ | Nₐ>3x10³ | Nₐ>3x10³ | Nₐ>3x10³ | Nₐ7.0x10⁶ | Nₐ2.1x10⁷ | Nₐ< 1.5 x10² |
| *Vc* = viable count *Na* = number of cfu/ml in the test mixture; NOTE: The BSEN 1276 standard dictates that colony counts of less than 15; are represented by < 1.5 x 10² and that counts of over 300 be presented as > 3 x 10³. | | | | | | | | |

| **Summary threshold results, *P. aeruginosa*** | | | | | **Summary threshold results, *S. aureus*** | | | |
|---|---|---|---|---|---|---|---|---|
| **E12 Concentration % (v/v)** | | **Reduction in Viability *Pseudomonas aeruginosa*** | | **of E12 Concentration % (v/v)** | | | **Reduction in Viability of *Staphylococcus aureus*** | |
| 0 | | >1x10⁸ | | 0 | | | >1x10⁸ | |
| 100 | | > 6 log | | 100 | | | > 5 log | |
| 66.67 | | > 6 log | | 66.67 | | | > 1 log | |
| 50 | | > 6 log | | 50 | | | - | |
| 33.33 | | > 6 log | | 33.33 | | | - | |
| 25 | | > 6 log | | 25 | | | - | |
| 20 | | > 6 log | | 20 | | | - | |
| 10 | | > 6 log | | 10 | | | - | |
| 5 | | > 6 log | | 5 | | | - | |

| **Test Results BSEN 1276, *E. hirae*** | | | | | | | | |
|---|---|---|---|---|---|---|---|---|
| **Bacterial Dilution** | **Neat** | **10⁻¹** | **10⁻²** | **10⁻³** | **10⁻⁴** | **10⁻⁵** | **10⁻⁶** | **10⁻⁷** |
| **Untreated control** | V_{c}>300 | V_{c}>300 | V_{c}>300 | V_{c}>300 | V_{c} 152 | V_{c} 19 | V_{c} 3 | V_{c}0 |
| | Nₐ>3x 10³ | Nₐ>3x 10³ | Nₐ>3x 10³ | Nₐ>3x 10³ | Nₐ 1.52 x 10⁵ | Nₐ 1.9 x 10⁶ | Nₐ<1.5x10² | Nₐ<1.5x10² |

| **E12 Concentration % (v/v)** | | | | | | | | |
|---|---|---|---|---|---|---|---|---|
| **100** | V_{c} 0 | V_{c} 0 | V_{c} 0 | V_{c} 0 | V_{c} 0 | V_{c} 0 | V_{c} 0 | V_{c} 0 |
| | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² | Nₐ<1.5x10² |
| **66.7** | V_{c} 27 | V_{c}>23 | V_{c} 1 | V_{c} 0 | V_{c} 0 | V_{c} 1 | V_{c} 0 | V_{c} 0 |
| | Nₐ2.7x10¹ | Na_{>}2.3x10² | Nₐ<1.5x10² | Nₐ<1.5x10² | Nₐ<1.5x10² | Nₐ<1.5x10² | Nₐ<1.5x10² | Nₐ<1.5x10² |
| **50** | V_{c} 111 | V_{c} 92 | V_{c} 18 | V_{c} 1 | V_{c} 0 | V_{c} 0 | V_{c} 0 | V_{c} 0 |
| | Nₐ1.11x10² | Nₐ9.2x10² | Nₐ1.8x10³ | Nₐ<1.5x10² | Nₐ<1.5x10² | Nₐ<1.5x10² | Nₐ<1.5x10² | Nₐ<1.5x10² |
| **33** | V_{c}>300 | V_{c}>300 | V_{c}>300 | V_{c} 32 | V_{c} 5 | V_{c} 1 | V_{c} 0 | V_{c} 0 |
| | Nₐ>3x 10³ | Nₐ>3x 10³ | Nₐ>3x 10³ | Nₐ 3.2x10⁴ | Nₐ<1.5x10² | Nₐ<1.5x10² | Nₐ<1.5x10² | Nₐ<1.5x10² |
| **25** | V_{c}>300 | V_{c}>300 | V_{c}>300 | V_{c} 56 | V_{c} 1 | V_{c} 0 | V_{c} 0 | V_{c} 1 |
| | Nₐ>3x 10³ | Nₐ>3x 10³ | Nₐ>3x 10³ | Nₐ 5.6x10⁴ | Nₐ<1.5x10² | Nₐ<1.5x10² | Nₐ<1.5x10² | Nₐ<1.5x10² |
| **20** | V_{c}>300 | V_{c}>300 | V_{c}>300 | V_{c} 90 | V_{c} 12 | V_{c} 3 | V_{c} 0 | V_{c} 1 |
| | Nₐ>3x 10³ | Nₐ>3x 10³ | Nₐ>3x 10³ | Nₐ 9.0x10⁴ | Nₐ<1.5x10² | Na<1.5x10² | Nₐ<1.5x10² | Nₐ<1.5x10² |
| **10** | V_{c}>300 | V_{c}>300 | V_{c}>300 | V_{c}>300 | V_{c} 34 | V_{c} 3 | V_{c} 1 | V_{c} 0 |
| | Nₐ>3x 10³ | Nₐ>3x 10³ | Nₐ>3x 10³ | Nₐ>3x 10³ | Nₐ 3.4x10⁵ | Nₐ<1.5x10² | Nₐ<1.5x10² | Nₐ<1.5x10² |
| **5** | V_{c}>300 | V_{c}>300 | V_{c}>300 | V_{c}>300 | V_{c} 124 | V_{c} 13 | V_{c} 2 | V_{c} 0 |
| | Nₐ>3x 10³ | Nₐ>3x10³ | Nₐ>3x 10³ | Nₐ>3x 10³ | Nₐ 1.24 x 10⁶ | Nₐ<1.5x10² | Nₐ<1.5x10² | Nₐ<1.5x10² |
| *V*c = viable count *N*a = number of cfu/ml in the test mixture NOTE: The BSEN 1276 standard dictates that colony counts of less than 15 are represented by < 1.5 x 10² and that counts of over 300 be presented as > 3 x 10³. | | | | | | | | |

| **Summary threshold results, *E. hirae*** | | | | | | | | |
|---|---|---|---|---|---|---|---|---|
| **E12 Concentration % (v/v)** | | | | **Reduction in Viability of *Enterococcus hirae*** | | | | |
| 0 | | | | >1x10⁸ | | | | |
| 100 | | | | > 5 log | | | | |
| 66.67 | | | | > 5 log | | | | |
| 50 | | | | > 4 log | | | | |
| 33.33 | | | | > 1 log | | | | |
| 25 | | | | > 1 log | | | | |
| 20 | | | | > 1 log | | | | |
| 10 | | | | - | | | | |
| 5 | | | | - | | | | |

| **Test Results BSEN 1276, *L. Monocytogenes*** | | | | | | | | |
|---|---|---|---|---|---|---|---|---|
| **Bacterial Dilution** | **Neat** | **10⁻¹** | **10⁻²** | **10⁻³** | **10⁻⁴** | **10⁻⁵** | **10⁻⁶** | **10⁻⁷** |
| **Untreated control** | V_{c} >300 | V_{c} >300 | V_{c} >300 | V_{c} >300 | V_{c} 289 | V_{c} 35 | V_{c} 4 | V_{c} 1 |
| | Nₐ>3x 10³ | Nₐ>3x 10³ | Nₐ>3x | 10³ Nₐ > 3 x 10³ | Nₐ 2.89 x 10⁶ | Nₐ 3.5 x 10⁶ | Nₐ< 1.5 x10² | Nₐ < 1.5 x10² |

| **E12 Conc. % (v/v)** | | | | | | | | |
|---|---|---|---|---|---|---|---|---|
| **100** | V_{c} 0 | V_{c} 1 | V_{c} 0 | V_{c} 0 | V_{c} 0 | V_{c} 0 | V_{c} 0 | V_{c} 1 |
| | Nₐ<1.5x10² | Nₐ<1.5x10² | Nₐ<1.5x10² | Nₐ< 1.5 x10² | Nₐ< 1.5 x10² | Nₐ< 1.5 x10² | Nₐ < 1.5 x10² | Nₐ<1.5x10² |
| **66.7** | V_{c} >300 | V_{c} >300 | V_{c} >300 | V_{c} >300 | V_{c} 97 | V_{c} 6 | V_{c} 0 | V_{c} 0 |
| | Nₐ>3x10³ | Nₐ>3x10³ | Nₐ>3x10³ | Nₐ >3x 10³ | Nₐ 9.7x 10⁵ | Nₐ < 1.5 x10² | Nₐ < 1.5 x10² | Nₐ<1.5x10² |
| **50** | V_{c} >300 | V_{c} >300 | V_{c} >300 | V_{c} 74 | V_{c} 2 | V_{c} 1 | V_{c} 0 | V_{c} 0 |
| | Nₐ>3x10³ | Nₐ>3x10³ | Nₐ>3x10³ | Nₐ 7.4x x 10⁴ | Nₐ < 1.5 x10² | Nₐ < 1.5 x102 | Nₐ < 1.5 x10² | Nₐ<1.5X10² |
| **33** | V_{c} >300 | V_{c} >300 | V_{c} >300 | V_{c} >300 | V_{c} 143 | V_{c} 15 | V_{c} 2 | V_{c} 0 |
| | Nₐ>3x10³ | Nₐ>3x10³ | Nₐ>3x10³ | Nₐ>3x 10³ | Nₐ 1.43 x 10⁶ | Nₐ 1.5 x 10⁶ | Nₐ < 1.5 x10² | Nₐ<1.5x10² |
| **25** | V_{c} >300 | V_{c} >300 | V_{c} >300 | V_{c} >300 | V_{c} 179 | V_{c} 14 | V_{c} 4 | V_{c} 0 |
| | Nₐ>3x10³ | Nₐ>3x10³ | Nₐ>3x10³ | Nₐ>3 x 10³ | Nₐ1.79 x 10⁶ | Nₐ < 1.5 x10² | Nₐ < 1.5 x10² | Nₐ<1.5x10² |
| **20** | V_{c} >300 | V_{c} >300 | V_{c} >300 | V_{c} >300 | V_{c} 252 | V_{c} 24 | V_{c} 0 | V_{c} 1 |
| | Nₐ>3x10³ | Nₐ>3x10³ | Nₐ>3x10³ | Nₐ>3 x 10³ | Nₐ 2.52 x 10⁶ | Nₐ 2.4 x 10⁶ | Nₐ < 1.5 x10² | Nₐ<1.5x10² |
| **10** | V_{c} >300 | V_{c} >300 | V_{c} >300 | V_{c} >300 | V_{c} 327 | V_{c} 14 | V_{c} 2 | V_{c} 0 |
| | Nₐ>3x10³ | Nₐ>3x10³ | Nₐ>3x10³ | Nₐ>3 x 10³ | Nₐ 3.27 x 10⁶ | Nₐ < 1.5 x10² | Nₐ < 1.5 x10² | Nₐ<1.5x10² |
| **5** | V_{c} >300 | V_{c}>300 | V_{c} >300 | V_{c} >300 | V_{c} >300 | V_{c} 30 | V_{c} 6 | V_{c} 0 |
| | Nₐ>3x 10³ | Nₐ>3x10³ | Nₐ>3x10³ | Nₐ>3x x 10³ | Nₐ>3x x 10³ | Nₐ 3.0x 10⁶ | Nₐ < 1.5 x10² | Nₐ<1.5x10² |
| *V*c = viable count *N*a = number of cfu/ml in the test mixture **NOTE:** The BSEN 1276 standard dictates that colony counts of less than 15 are represented by < 1.5 x 10² and that counts of over 300 be presented as > 3 x10³. | | | | | | | | |

| **Summary threshold results, *L. monocytogenes*** | | | | | | | | |
|---|---|---|---|---|---|---|---|---|
| **E12 Conc % (v/v)** | **Reduction in Viability of *Listeria monocytogenes*** | | | | | | | |
| **0** | >1x10⁸ | | | | | | | |
| **100** | > 5 log | | | | | | | |
| **66.67** | > 1 log | | | | | | | |
| **50** | | | | | | | | |
| **33.33** | | | | | | | | |
| **25** | | | | | | | | |
| **20** | | | | | | | | |
| **10** | | | | | | | | |
| **5** | | | | | | | | |

| **E12 Concentratio n % (v/v)*** | Gram Negative versus Gram Positive Results | | | | | | | |
|---|---|---|---|---|---|---|---|---|
| | **Bacteria** | | | | | | | |
| | **Gram Negative** | | | | **Gram Positive** | | | |
| | *Campylobacte r jejuni* | ***Escherichia coli*** | *Salmonella typhimurium* | *Pseudomonas aeruginosa* | *Staphylococcusaureus* | | *Enterococcus hirae* | ***Listeria monocytogenes*** |
| **0** | >1x10⁸ | > 1 x10⁸ | > 1 x10⁸ | > 1 x10⁸ | > 1 x10⁸ | | > 1 x10⁸ | > 1 x10⁸ |
| **100** | > 6 log | > 6 log | > 6 log | > 6 log | > 5 log | | > 5 log | > 5 log |
| **66.67** | > 6 log | ≥ 3 log | > 6 log | > 6 log | > 1 log | | > 5 log | > 1 log |
| **50** | > 6 log | > 2 log | > 6 log | > 6 log | | | > 4 log | |
| **33.33** | > 6 log | > 2 log | > 6 log | > 6 log | | | > 1 log | |
| **25** | > 6 log | < 1 log | > 3 log | > 6 log | | | > 1 log | |
| **20** | > 6 log | < 1 log | > 3 log | > 6 log | | | > 1 log | |
| **10** | > 6 log | < 1 log | > 1 log | > 6 log | | | | |
| **5** | > 5 log | < 1 log | | > 6 log | | | | |
| **NOTE:** Cells shaded yellow indicate the lowest concentration which induces a ~ 6 log order reduction (or greater) in bacterial viability. | | | | | | | | |
| **NOTE: E12 is commercially referred to as CeBeC "C-Activ-12" Natural Sanitiser** | | | | | | | | |
| **NOTE: 100% (v/v) represents 0.5% base dilution** | | | | | | | | |

### Summary of log reduction threshold expressed in terms of C-Activ-12 concentration

The current formulation is therefore effective at a 5 log reduction level against all seven test organisms (designated strains) at a MIC concentration of ~0.5% (v/v). Due to the presence of non-flavonoid natural compounds in the sourced complex, the final pure flavonoid concentration is ~ 0.25% (v/v).

### Efficacy Testing of CeBeC Natural Biocides Against Planktonic Campylobacter and Campylobacter Isolates in Bio-films ― University College Dublin Dr Stephen O'Brien

96 well plates were used to test a range of CeBeC Natural biocides against *Campylobacter jejuni* Type strain NCTC 11168 and Wild Type *Campylobacter jejuni* (isolated from a poultry sample). All biocides were tested at a range of concentrations. Where a range of working concentrations were given, the highests recommended concentration was considered the 100% working concentration. For example, if the working was recommended for use at 0.5-1%, a 1% v/v solution in sterile water was considered the 100% working concentration. A range of concetrations were prepared representing 200, 100, 50, 10, 1, 0.1 and 0.01 % of the biocides working concentration and added to a 96 well plate. The plates were incubated microaerobically for 24h at 41.5°C. A pin replicator was then used to trasfer samples from each well to a 96 well plate filled mCCDA agar. The mCCDA plates were incubated microaerobically at 41.5°C for 24h and examined for growth. All strains were repeated in triplicate. The *Campylobacter jejuni* strains were also tested in a biofilm. This was achevied by addeding overnight cultures to the 96 well plate and incubating them at 41.5°C for 24 h. The media was carefully removed from the each well and replaced with 100 µl of fresh media again followed by incubation at 41.5°C for 24 hours. This process was repeated a total of 3 times. The formation of a biofilm was confirmed using a Crystall Violet assay. The biofilms were then tested with the various biocide concentrations as described above. More detail on preparing the assay has been appended to the end of this document. Results were recorded for wells where growth was observed.

**Results** - No cell growth was observed in any of the biocides when used in their recommended concentration ranges. There was no difference observed between the ability of the *C. jejuni* Type strain or Wild Type strain to surviving biocide exposure. All of the biocides were effective at their recommended working concentration on planktonic *C. jejuni* and those in a biofilm. The biocides were consistantly effective at killing Log₁₀ 5 CFU of *C. jejuni* when used at 50% of their recommended working concentration. The full results are presented in Table I, Table II, Table III and Table IV on pages 4 to 6 of this report.

**Protocol for MIC of biocides** - Inoculum Grow strains overnight (16-18 h) in 10 ml Mueller-Hinton broth with *Campylobacter* growth supplement at 41.5°C with shaking (200 rpm).Strains should reach ≥10⁹ CFU/ml.Dilute in Mueller-Hinton with *Campylobacter* growth supplement broth to give approx 10⁷ CFU/ml.

Add 100 µl of approx 10⁷ CFU/ml to 10 ml double-strength (ds) Mueller-Hinton broth with *Campylobacter* growth supplement to give an inoculum of approx 10⁵ CFU/ml (when 100 µl is added to each well in the plate the inoculum per well is approx 10⁴ CFU).

Biocide Determine the 100% working concentration of each biocide. Example - If the recommended working concentration is 0.5-1% of the concentrate provided (consider 100% working concentration to be 1% solution of the concentrate).Prepare double-strength solutions of the biocides in sterile distilled H₂O (i.e. for 200% working concentration in the plate make a 400% working concentration solution). Example - If the working concentration is a 1% solution of the concentrate; prepare 4% solution of the concentrate for a 400% working concentration. If the sterility of the 400% working concentration may be in doubt (possible if using a detergent not a disinfectant) then filter sterilise before proceeding with the dilutions. Prepare dilutions of the biocides to give the final % working concentrations according to the screening plates (see table below). Example - to give enough of each concentration for approximately 50 plates (50 strains), given 100 µl in 3 wells per plate.

| **For a working concentration of 1%** | | | |
|---|---|---|---|
| For final % working concentration | Prepare % working concentration | Biocide | Sterile distilled H₂O (ml) |
| 200 | 400 | 1.6 ml Biocide concentrate | 38.4 |
| 100 | 200 | 15 ml of 400% (above) | 15.0 |
| 50 | 100 | 12 ml of 200% (above) | 12.0 |
| 10 | 20 | 4 ml of 100% (above) | 16.0 |
| 1 | 2 | 2 ml of 20% (above) | 18.0 |
| 0.1 | 0.2 | 2 ml of 2% (above) | 18.0 |
| 0.01 | 0.02 | 2 ml of 0.2% (above) | 18.0 |
| 0 | 0 | 0 | 20.0 |

Screening plates - For all strains against all biocides use the final working concentrations below. Use multiple biocides per plate and one strain per plate (this is to reduce cross-contamination of strains). Use a minimum of N=3 per biocide. Add to 3 wells per row 100 µl of the % working concentrations

Incubation - Add 100 µl inoculum at ~10⁵ CFU/ml to each well. Incubate plates microaerobically for 24h at 41.5°C. Use a pin replicator to trasfer samples from each well to a 96 well plate filled mCCDA agar. Incubate mCCDA plates were incubated microaerobically at 41.5°C for 24h and examined for growth. All strains were repeated in triplicate.

### Results & Minimum Inhibitory Concentrations of Strains

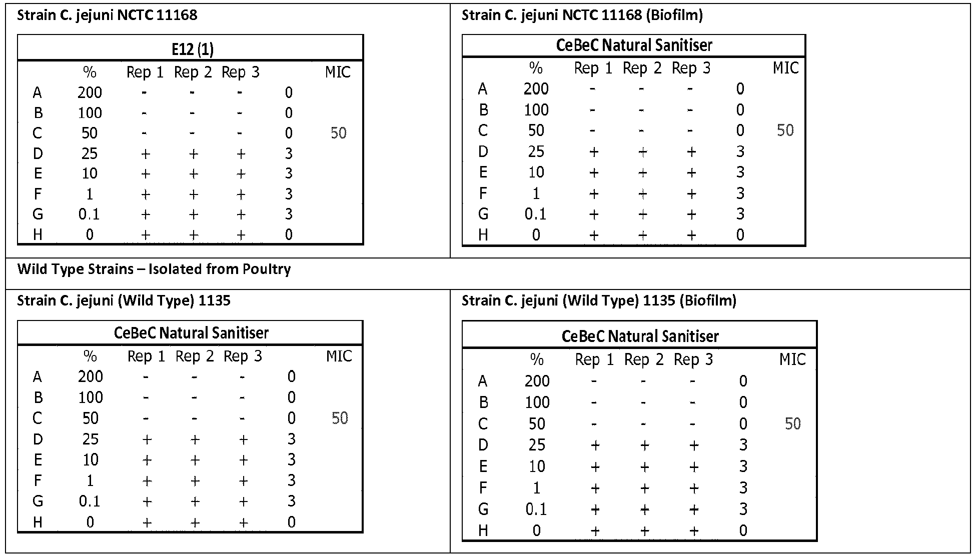

### Epidemiology Study On Efficacy of CeBeC Natural Biocides Against Campylobacter Isolates from Irish Broiler Farms - Dr Emer O'Mahony University College Dublin

**Tolerance** - Twenty-five *Campylobacter* isolates from Irish broiler farms and two reference strains were examined for tolerance to formulations of C-ACTIV-12, a commercial mixture of generally recognized as safe (GRAS) approved extracts. **Isolate selection and growth conditions** - Isolates were cultured and confirmed to the genus and species levels as previously described. The *C. jejuni* Type strain NCTC 11168 and a *C. jejuni* strain (denoted 1135) isolated from a poultry sample were also included. The isolate collection was stored at -80°C on Protect cryobeads (Technical Service Consultants Ltd) containing 80% glycerol, for subsequent testing. When required, *Campylobacter* isolates were cultured from the frozen stock on Mueller-Hinton agar plates (MHA CM337, Oxoid) supplemented with 5% (v/v) lysed horse blood (TCS Biosciences), under microaerophilic conditions (10% CO₂, 5% O₂ and 85% N₂) using a GENbox MicroAir (bioMérieux) at 37°C for 24 h. Isolates were sub-cultured onto Columbia agar (Oxoid, UK), containing 5% (v/v) lysed horse blood, and incubated under microaerophilic conditions at 41.5 °C for 48 h. **Biocide tolerance testing** - Strains were grown under microaerophilic conditions overnight (16 -18 h) in 5 ml Mueller Hinton broth (MHB, Oxoid) at 41.5 °C to reach approximately 10⁷ CFU/ml. From this suspension, 100 µl was transferred into 10 ml double strength MHB (dsMHB) to give an inoculum of approximately 10⁵ CFU/ml. A stock solution (4 x recommended working concentration) of each biocide formulation was prepared. Dilutions (100 µl) of each formulation were prepared in triplicate using sterile distilled H₂O in sterile 96-well plates (Sarstedt) and 100 µl of the (approximately) 10⁵ CFU/ml inoculum was added to each well (giving a final inoculum of approximately 10⁴ CFU/well) . Plates were incubated microaerophilically for 24 h at 41.5 °C. A 96 pin replicator (Nunc^{®}) was used to transfer approximately 1 µl from each well to *Campylobacter* blood-free selective agar base (CCDA, CM0739, Oxoid), supplemented with CCDA selective supplement (SR0155, Oxoid) in 96 well plates. These plates were incubated microaerophilically for 24 h at 41.5 °C. The minimum inhibitory concentration (MIC) was considered as the lowest concentration with no visible growth present. Results are shown below. **Results** - 100% (n= 27) of *Campylobacter* isolates studied were susceptible to all C-ACTIV-12 formulations at ≤ 100% of the recommended working concentration. 100% of *Campylobacter* isolates studied were susceptible to C-ACTIV-12 at ≤ 50% of the recommended working concentration.

| MICs of each formulation of the biocide C-ACTIV-12¹ | | | | | | | |
|---|---|---|---|---|---|---|---|
| **Strain** | **Source** | **Species** | **C-ACTIV-12** | **Strain** | **Source** | **Species** | **C-ACTIV-12** |
| Se 2 | Breeder faeces | *C*. *jejuni* | 50 | Se 185 | Breeder faeces | *C*. *coli* | 50 |
| Se 5 | Breeder faeces | *C*. *jejuni* | 50 | Se 226 | Broiler faeces | *C*. *jejuni* | 50 |
| Se 19 | Breeder faeces | *C*. *jejuni* | 50 | Se 241 | Breeder faeces | *C*. *coli* | 50 |
| Se 43 | Breeder faeces | *C*. *jejuni* | 50 | Se 246 | Breeder faeces | *C*. *coli* | 50 |
| Se 46 | Breeder faeces | *C*. *coli* | 50 | Se 266 | Breeder faeces | *C*. *coli* | 50 |
| Se 50 | Breeder faeces | *C*. *coli* | 50 | Se 279 | Breeder faeces | *C*. *coli* | 50 |
| Se 54 | Breeder faeces | *C*. *coli* | 0.01 | Se 311 | Breeder faeces | *C*. *coli* | 50 |
| Se 77 | Broiler faeces | *C*. *jejuni* | 50 | Se 316 | Breeder faeces | *C*. *coli* | 50 |
| Se 83 | Adj broiler faeces | *C*. *jejuni* | 50 | Se 330 | Breeder faeces | *C*. *coli* | 50 |
| Se 84 | Adj broiler faeces | *C*. *jejuni* | 10 | Se 341 | Breeder faeces | *C*. *jejuni* | 50 |
| Se 85 | Adj broiler faeces | *C*. *jejuni* | 50 | Se 366 | Broiler faeces | *C*. *jejuni* | 50 |
| Se 86 | Adj broiler faeces | *C*. *jejuni* | 50 | Se 376 | Broiler faeces | *C*. *jejuni* | 50 |
| Se 96 | Broiler faeces | *C*. *jejuni* | 50 | 1135 | Poultry | *C*. *jejuni* | 50 |
| | | | | NCTC 11168 | Human faeces | *C*. *jejuni* | 50 |
| Table Footnotes: ¹MIC is reported as % of recommended working concentration; = 1% = 99 parts water plus 1 part C-ACTIV-12 | | | | | | | |

**Summary of initial findings** - In the early stages of these investigations, efforts were concentrated on two zoonotic pathogens namely verocytoxigenic *Escherichia coli* 0157 and *Salmonella* species, along with the recently described powdered infant formula pathogen *Cronobacter* species. All isolates analysed (in each case > 100 were selected from strains recovered during various epidemiological studies) were found to be susceptible to biocides in common use in the meat and PIF industries. Some of the genera appeared to be more susceptible compared with others, as in the case of *Cronobacter.* Attempts to develop tolerant phenotypes were marginally successful and where this was generated, the phenotype was unstable. As a means of modelling the increased tolerance to biocide-active compounds, a small number of isolates were challenged with increasing concentrations of actives such as triclosan, chlorhexidine and benzalkonium chloride. Highly-tolerant mutant phenotypes were obtained and these are currently being studied in detail, using a dedicated technical pipeline, described above. It was decided that it would be of interest to include the food-borne zoonotic pathogen *Campylobacter* and to evaluate its susceptibility/tolerance to the same panel compounds. Once again, it could be shown that *Camptlobacter* was susceptible. However, as the panel of compounds used could be described as synthetic, the availability of C-Activ-12 presented the opportunity to re-evaluate the responses of these bacteria to this natural biocide. Initially planktonically-growing *campylobacter* were tested and found to be completely susceptible to C-Activ-12 at concentrations ranging from 0.5 through 1% (w/v). Similarly when these same cells were part of a biofim, C-Active-12 remained effective over the latter concentration range. These studies were subsequently extended to include *Cronobacter, Salmonella* and *E. coli* (as outlined above) and in each case the bacteria tested were found to be susceptible to the natural biocide, being bacteriocidal in all cases. In the current project proposal, this inhibitory effect will be characterised in detail, to provide a means of understanding the mode of action. In this way it is envisaged that the C-Activ-12 compound can be modified to enhanve its bacteriocidal effects on these and other pathogens (preliminary data suggests biocidal activity against, important clinical pathogens such as *Acinetobacter baummanii* and *Clostridium difficile,* along with a number of canine dental pathogens also). C-Activ-12 is a novel natural biocide. The formulation ingredients are FDA GRAS approved comprising organic acids and natural fruit extracted polyphenols. The broad spectrum activity of the formulation resulted it its inclusion in a University College Dublin Government funded independent study under the FIRM (see hereunder) research program. The program studies the effectiveness of products used to manage micro-organisms/pathogens prevalent in the food sector, comparing effectiveness & testing against isolated strains. To date CeBeC's C-Activ-12 has killed all organisms tested under FIRM, this study is on-going. The Irish Department of Agriculture and Food's Food Institutional Research Measure (FIRM) is funded under the National Development Plan 2007-2013. FIRM is the primary national funding mechanism for food research in third level colleges and research institutes. FIRM is a public good competitive programme whereby multi-disciplinary teams from two or more institutions usually carry out the research projects. Research outputs are communicated to industry by a dedicated dissemination team known as RELAY. FIRM aims to develop public good technologies that will underpin a competitive, innovative and sustainable food manufacturing and marketing sector. The programme is creating a base of knowledge and expertise in generic technologies that will support a modern, consumer-focused industry and build Ireland's capacity for R&D. A key output of the FIRM is highly trained young researchers at PhD and postdoctoral level, with specialist skills particularly relevant to the Irish food sector. The following pages provide an overview of the effectiveness of CeBeC's C-Activ-12 in killing micro-organisms. Standard testing protocols were first applied by AIT CBBR under British & European Food Grade Sanitiser Standard BS EN 1276, requiring a log 5 reduction from a log 7 to a log 2. The activity of C-Activ-12 highlighted a greater than log 6 reduction from a log 8. Based on the results under BS EN 1276, UCD added the product under the FIRM study whereby pathogens were isolated from food production environments. The rationale behind this approach is that most standards rely on ATCC & NCTC cultures which are more than often easier to kill than isolates. CeBeC's natural food based biocide has proven effective against strains isolated from growing & processing facilities and against strains isolated from humans. The following results provide an overview of efficacy under BS EN 1276 strains whilst providing more detail on activity against Pseudomonas.

| **Comparison of Active Substances (Biocidal Compounds) UCD FIRM Study -** Sample size (n=74). 74 strains involved In this screening. | | | | |
|---|---|---|---|---|
| Compounds | Unit | MIC | Stdev | Range |
| Eugenol | ul/ml | 0.86 | 0.38 | 0.25-2 |
| Benzoic Acid | ug/ml | 810.51 | 398.91 | 247.5-1320 |
| Trans-cinnaldeheyde | ul/ml | 0.43 | 0.19 | 0.2-0.8 |
| Cininamic Acid | ug/ml | 95.12 | 44.09 | 23.44-140.65 |
| a-Terpineol | ul/ml | 2.43 | 1.64 | 0.53-7.59 |
| Geraniol | ul/ml | 8.29 | 4.50 | 0.94-15 |
| Thymol | ug/ml | 85.73 | 52.511 | 19.69-315 |
| E12/C-Activ-12 | ul/ml | 2.92 | 1.21 | 0.625-10 |
| | | | | |

| Compounds | Unit | MBC | Stdev | Range |
|---|---|---|---|---|
| Eugenol | ul/ml | 0.99 | 0.33 | 0.25-2 |
| Benzoic Acid | ug/ml | 920.79 | 483.34 | 165-1320 |
| Trans-cinnaldeheyde | ul/ml | 0.50 | 0.29 | 0.05-0.8 |
| Cininamic Acid | ug/ml | 119.40 | 52.01 | 23.44-421.88 |
| a-Terpineol | ul/ml | 2.84 | 2.31 | 0.42-7.59 |
| Geraniol | ul/ml | 9.46 | 5.39 | 0.94-22.5 |
| Thymol | ug/ml | 121.38 | 66.39 | 4.92-315 |
| E12/C-Activ-12 | ul/ml | 2.95 | 2.16 | 0.625-10 |
| **FIRM Study - Market Place Biocidal Product Comparative Effectiveness - E12 highlighted = C-Activ-12** | | | | |

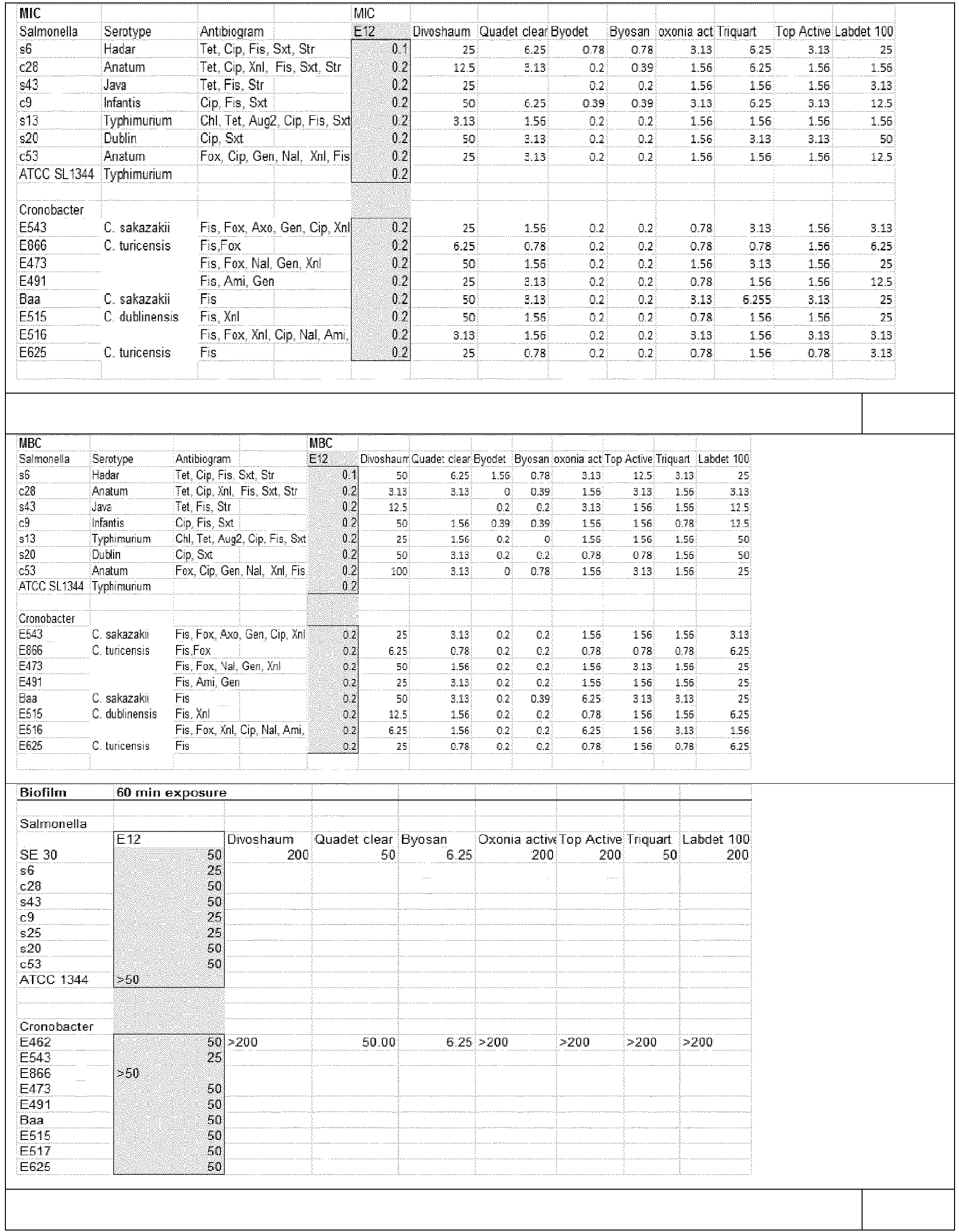

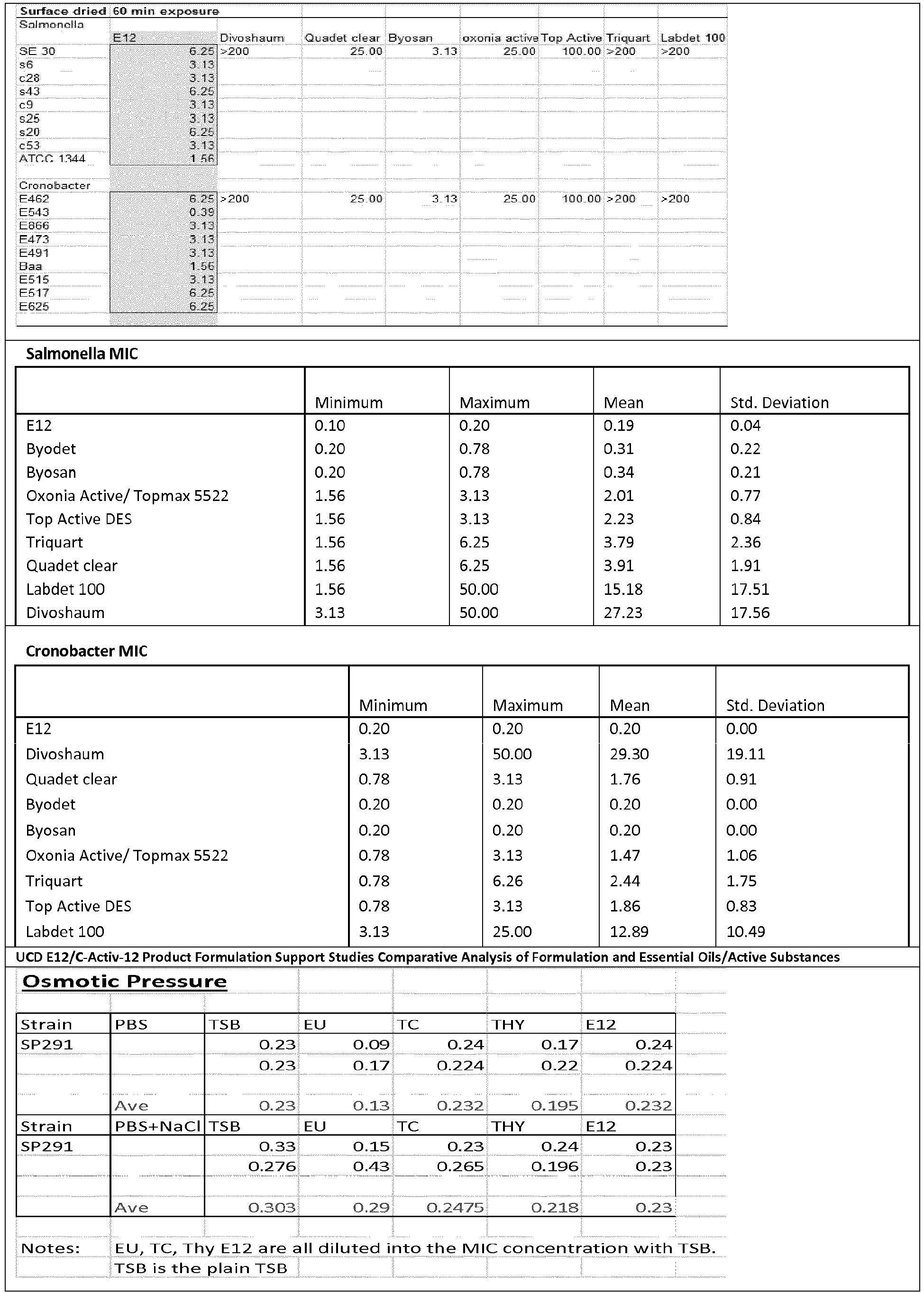

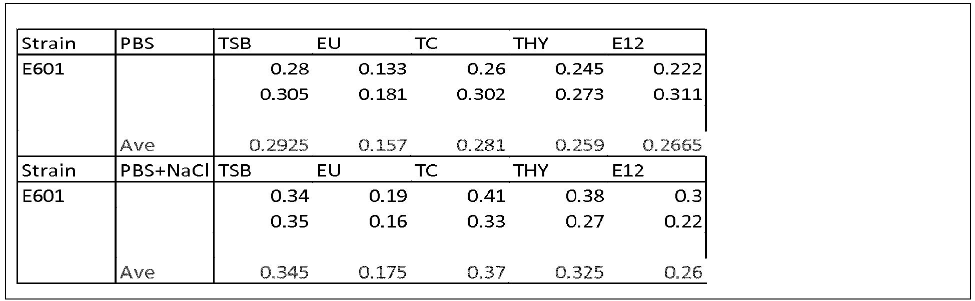

### Fresh Produce Decontamination - Product Comparison Study University of Padova, Italy. -Study 1-Salad Wash Test Padova June 2011

In comparison: 2 salads ("Biological" and "Conventional"). Biological is from organic farming and it is always pre treated in-field with a mix of plant growth- protecting bacteria and fungi. Conventional is grown with pesticides and chemical fertilizers. Both salads were spiked with a culture of Escherichia coli resistant to Kanamycin by sprinkling them with a cdosage that conveyed about 3 x 10⁷ cells per gram). 125 grams were treated for each salad. Salads were incubated 24 h at 37 ° C in sealed envelopes. 4 aliquots of 25 g were taken and washed with 3 different washing for 10 minutes by stirring in 1500 ml of: 1) water, 2) 30 ppm hypochlorite, 4) 1% CebecC-Activ-12 + surfactant. Salads were rinsed 3 minurtes in 1 volume of water, drained and transferred to stomacher bags, to which added 150 ml of physiological solution were added. Bags were traeted for 2 minutes at 230 rpm in the stomacher apparatus. The resuspension was plated on two different media (dilutions from 10⁰ a 10⁻⁷ carried out in duplicate) A) PCA (Plate Count Agar) for total count. Incubation at 22 ° C B) agar LB + Kanamycin 30 ug /ml for Escherichia coli. Incubation at 37 ° C. Counts at 24 hours, further incubation for 6 days.

| | CFU per g fw salad | | Fold reduction over water* |
|---|---|---|---|
| **LB Km 37°C** | Mean | St Dev | |
| Bio Salad Water | 2,79 x 10⁶ | 1,06 x 10⁶ | |
| Bio Salad Chlorine | 3,51 x 10⁵ | 1,06 x 10⁵ | 7,95 |
| Bio Salad C-activ-12 | 4,92 x 10⁵ | 1,87 x 10⁵ | 5,67 |
| Conventional Salad Water | 2,43 x 10⁶ | 6,36 x 10⁵ | |
| Conventional Salad Chlorine | 9,90 x 10⁵ | 2,12 x 10⁵ | 2,45 |
| Conventional Salad C-activ-12 | 5,70 x 10⁴ | 4,24 x 10³ | 42,63 |

| | CFU per g fw salad | | Fold reduction over water |
|---|---|---|---|
| **PCA 22°C** | Mean | St Dev | |
| Bio Salad Water | 8,37 x 10⁷ | 1,57 x 10⁷ | |
| Bio Salad Chlorine | 1,65 x 10⁶ | 2,97 x 10⁵ | 50,73 |
| Bio Salad C-activ-12 | 4,50 x 10⁶ | 2,12 x 10⁶ | 18,60 |
| Conventional Salad Water | 2,49 x 10⁸ | 7,21 x 10⁷ | |
| Conventional Salad Chlorine | 2,88 x 10⁷ | 1,70 x 10⁶ | 8,65 |
| Conventional Salad C-activ-12 | 5,10 x 10⁴ | 4,24 x 10³ | 4882,35 |

| | | | |
|---|---|---|---|
| *Fold reduction over water show many folds are bacteria abated, compared to water (=CFU with water /CFU with product under study) | | | |

### Fresh Produce Study 2 - Antimicrobial activity of chlorine, iodine, and C-Activ-12 on Salmonella

***enteritidis* and *Escherichia coli*** - The microorganisms were cultured in the presence of varying concentrations of chlorine, iodine and C-Activ 12 for time intervals of different lengths before being transferred to nutrient medium. **Products tested** - Amuchina ^{®} whose active ingredient is sodium hypochlorite, 1.15 g /100 ml (equal to 1.1 g of active chlorine). Sodium hypochlorite dilutions were prepared to give final concentrations of: 0, 10, 20, 30, 40, 50, 75, 100, 150, 200, 250 and 300 ppm. Poseidon-500 ^{®}, Poly Vinyl Pyrrolidone iodine, 5% solution. - Dilutions of iodine were made to give final concentrations of 0, 10, 20, 30, 40, 50, 75, 100, 150, 200, 250, 300, 350, 400, 450 and 500 ppm.

CeBeC C-activ-12 (+ surfactant) - The following increasing concentrations were tested: 3 µl/ml (0.3%), 6 µl/ml (0.6%) , 10 µl/ml (1%), 15 µl/ml (1.5%), 30 µl/ml (3%), 50 µl/ml (5%). Preparation of inocula *E. coli* ATCC 8739 and a wild strain of *Salmonella enteritidis* isolated from a hospitalized patient were tested. - All experiments were performed on cultures of early logarithmic phase. Flasks containing 25 ml of Nutrient Broth (Merck) were inoculated and incubated with agitation for 18 hours at 37 ° C. The optical density of cultures was adjusted by dilution with sterile Nutrient Broth to produce a density of 2 x10⁷ CFU ml⁻¹ **Determination of bacteriostatic activity of the compounds.** - Tests were set up using disposable multiwell plates (96 wells, 300µl, conical bottom). The wells were filled with 60µl solution of sodium hypochlorite or iodine, 60µl of the bacterial suspension at a concentration of 2x 10⁷ CFUml-1, and 120 µl of Nutrient Broth. The plates were closed and incubated at 37 ° C for 24 hours with shaking. The inhibitory effect of iodine and sodium hypochlorite was assessed by the optical density readings (600 nm) of the wells at different time intervals T_0 (immediately after inoculation) T_6 (6 hours after inoculation), T_24 (24 hours after inoculation) using a microwell plate reader (Multiskan EX, Thermo). All experiments were performed in duplicate. **Determination of bactericidal activity of the compounds** - After 6 and 24 hours of exposure to the antimicrobials, 20 µl of bacterial suspensions were transferred into 180 µl of Nutrient Broth. From this first dilution, an aliquot of 20 µl was transferred to 180 µl of Nutrient Broth to obtain the dilution of the solutions of antimicrobials and a reduction of the initial inoculum to about 1x10⁴ microorganisms. The inoculated microwell plates were incubated at 37° C, stirred for 24 hours before determining the microbial growth using optical density readings.

**Collection of the results** - The MIC (minimum inhibitory concentration) was defined as the lowest concentration of antimicrobial at which there was no growth of microorganisms after 24 hours. The MBC (minimum bactericidal concentration) was defined as the lowest concentration of antimicrobials after which there was no re-growth of microorganisms in the medium upon the above dilution and transfer. **Variation of the protocol to suit the properties of C-Activ-12** The spectrophotometrical reading of the bacterial cultures turned out to be affected by the brown colour of C-activ-12, which interfered in the OD600 absorbance. Therefore the initial set up of the multiwell plates was substituted with an incubation in 100-ml flasks using 24 ml of Nutrient Broth and assessment of colony forming units after exposure to the different concentrations of the product for the three time intervals. In practice 70 µl, 140 µl, 240 µl, 360 µl, 720 µl, 1200 µl of C-Activ-12 were added to the flasks along with 6 ml of the bacterial suspension at a concentration of 2x10⁷ CFU/ml. The flasks were incubated at 37°C in agitation for 24 hours. After 0, 6 and 24 hours of exposure, 1 ml of each bacterial suspension was transferred into PBS buffer and serially diluted. Volumes of 1 ml for each dilution were seeded in triplicate for inclusion in a growth medium (Nutrient Agar). The plates were incubated at 37°C for 24 hours. **Results** - Chlorine and iodine display straight bactericidal activity (MIC coincides with MBC).

The two microorganisms show a different sensitivity only with respect to iodine and C-Activ 12. *Escherichia coli* was more resistant, with MBC values higher than those of *Salmonella enteritidis.* -The MBC for chlorine is 150 ppm for both organisms (Table 1). Iodine has less bactericidal activity than chlorine (Table 1) showing higher MBC_24 concentration: 450 ppm and 500 ppm respectively for *Salmonella* and *E. coli.* The MBCs for C-Activ-12 at 6h and 24h for *Salmonella enteritidis* and *Escherichia coli* have different values for the two organisms tested (Tables 2 and 3). *Salmonella enteritidis,* in fact, shows a MBC to T_6 of 50 µl/ml, while T_24 µl/ml is 15. *Escherichia coli* however, shows a higher resistance: in fact, the MBC T_6 is> 50 µlml-1, while the MBC in T_24 µl/ml is 30. As regards C-Activ 12 as Tables 2 and 3 show, there are interesting phenomena, for *E.coli* the 1,5% concentration at 24h exposure brings about a higher than six log reduction (from 9,60 to 3.35) and higher concentrations (3% onwards) yield a complete abatement of vitality. For Salmonella the >6 log reduction is seen already at 6 h of exposure with the 3% solution and the fully clean plates are seen at 24h also with the 1,5%. The mode of action appears to rely on a time-dependent mechanism as the 6h exposure shows effects that reach higher severity at 24h. An noteworthy parallel observation is that the yellowish colour of the C-Activ-12 solution tends to fade out in the bacterial cultures in a manner that appears inversely proportional with their vitality. I.e. the more they die the less C-Activ 12 seems to be left in solution. This suggests a gradual partitioning of the colour-absorbing compounds into the bacterial cells for which compartments (e.g. membranes) there could be a chemical affinity. **Effectiveness of C-Activ-12 against**

### Powdery Mildew (Plant Fungus) University of Padova Italy - Activity of C-Activ-12 against Plant Pathogen PSA - University of Padova Italy

The results of the MIC activity of C-Activ-12 on P. syringae pv. actinidiae and the quantification of its bacteriocidal effect upon exposure are provided and indicate that the product is very active up to a concentration of 0.125% to 0.0625 % depending on the strain. It has a marked acidifiying activity but its biocidal effects are independent from the pH factor (see controls with HCl and NaOH). A 5 min contact of cells with a 0.5% concentration abates their viability by 43-fold.

**PSA Study - MIC Assay** - Bacteria tested: *Pseudomonas syringae* pv. *actinidiae* strain 8.43, *Pseudomonas syringae* pv. *actinidiae* strain 8.43a , Source: Marco Scortichini CRA Rome; Growth medium: Nutrien Broth + 5% Sucrose (NSB medium); Pre-inoculum: Exponential phase liquid pre-cultures in 10 ml (having reached a viable cell count of 0,7 x 10⁷ CFU/ml); Product under study: C-Activ-12 - Dilutions tested (and corresponding concentrations): 1:100 (1%); 1:200 (0.5%); 1:400 (0.25%); 1:800 (0.125%), 1:1600 (0.0625%); 1:3200 (0.0312%); Procedure Tests were done in 50 ml falcon tubes containing 10 ml of NSB medium supplemented with decreasing amounts of C-Activ-12 to yield the above concentrations. Tubes were inoculated with 10 µl of bacterial preculture. As control tubes without C Activ-12 were set up. Incubation: 28°C stirring; Measurement : Bacterial growth (turbidity) was inspected after 18h and after 48h; Other Controls and their rationale In addition to the above described standard tests, a number of controls was run. Control of C-Activ-12 sterility. In order to rule out that bacteria other that the ones under study could grow as a result of their cells or spores being possibly present in the C-Activ-12 stock itself. A series of tubes with the same C-Activ-12 dilutions in NSB was done, to which the inoculum of *P.syringae* pv. *actinidiae* was not added. Control of acidity effects. In order to distinguish between inhibitory effects of the C-Activ-12 compounds and those brought about by the low pH consequent to the addition of C-Activ-12 to the medium, the pH values corresponding to the different dilutions in NSB medium were measured prior to running the assays. Afterwards, the exact amounts of defined HCl solutions (1N and 0.1 N) to be added to NSB medium were determined in separate batches using a pH meter. With these data a series of tubes was set up in which those pH values were achieved by adding the required amounts of filter-sterilized (0.22 µm) HCl solutions. Aliquots to be added to the 10 ml cultures were in the range of 50 to 400 µl de3pending on the target pH. In these tubes therefore no C Activ-12 was added while the effects on bacterial growth of the corresponding HCl-generated pH values were assessed. Control of C-Activ-12 effects at re-neutralized pH. In order to uncouple the growth inhibiting effect due to the C Activ-12 mechanism to that given by the acidity that it conferred to the solution, a series of tubes with the above mentioned dilutions were corrected back to near-neutral pH using filter-sterilized NaOH 0.1N or 1 N solutions. The exact amounts to be added had been previously calculated experimentally by serial additions of NaOH to separate batches of NSB using a pH meter. **Results =**

| **Strain** | **C-Activ-12 concentration** | **pH*** | **Growth at time = 18 h** | **Growth at time = 48 h** |
|---|---|---|---|---|
| 8.43 | 0 | 7.08 | +++ | +++ |
| 8.43 | 1% | 3.75 | - | |
| 8.43 | 0.5 % | 4.27 | - | - |
| 8.43 | 0.25 % | 4.91 | - | - |
| 8.43 | 0.125 % | 5.58 | - | - |
| 8.43 | 0.0625 % | 6.38 | - | - |
| 8.43 | 0.00312% | 6.75 | - | +++ |
| 8.43a | 0 | 7.08 | +/- | +++ |
| 8.43a | 1% | 3.75 | - | - |
| 8.43a | 0.5 % | 4.27 | - | - |
| 8.43a | 0.25 % | 4.91 | - | - |
| 8.43a | 0.125 % | 5.58 | - | - |
| 8.43a | 0.0625 % | 6.38 | - | +++ |
| 8.43a | 0.00312% | 6.75 | - | +++ |

| a) sterility Control of C-Activ-12 alone without *P. syringae* pv. *actinidiae* | | | | |
|---|---|---|---|---|
| None | 1 % | 3.75 | - | - |
| None | 0.5 % | 4.27 | - | - |
| None | 0.25 % | 4.91 | - | - |
| None | 0.125 % | 5.58 | - | - |
| None | 0.0625 % | 6.38 | - | - |
| None | 0.00312% | 6.75 | - | - |

| b) pH effect without C-Activ-12 (obtained by HCl) | | | | |
|---|---|---|---|---|
| 8.43 | | 3.74 | - | - |
| 8.43 | | 4.34 | - | - |
| 8.43 | | 4.60 | - | - |
| 8.43 | | 5.65 | - | - |
| 8.43 | | 6.56 | - | - |
| 8.43 (No HCl) | | 7.08 | +++ | +++ |

| c) C Activ-12 activity in broth re-neutralized with NaOH | | | | |
|---|---|---|---|---|
| 8.43 | 1% | 6.97 | - | - |
| 8.43 | 0.5 % | 6.92 | - | - |
| 8.43 | 0.25 % | 6.91 | - | - |
| 8.43 | 0.125 % | 6.90 | - | - |
| 8.43 | 0.0625 % | 6.90 | - | - |
| 8.43 | 0.00312% | 6.95 | - | +++ |
| * the pH indicated is meant as the one at the beginning of the test. The values are deduced from parallel pH meter tests with given amounts of medium and C-Activ-12, or HCl, or NaOH as appropriate. | | | | |
| Ranking: - : no growth; +/- :very slight turbidity; +++ : full growth (OD600 > 0.8). | | | | |
| Note: at 1% and 0.5% dilutions the C-Activ-12 in NSB medium presents a slight presence of flocculating material, this is independent from the bacterial presence as it occurs also in the tubes without bacteria. | | | | |
| Comments - MIC: The minimum inhibitory concentration of C-Activ-12 resulted between 0.0625 % (MIC for strain 8.43) **and** 0.125 % (MIC for strain 8.43a). | | | | |
| -The C-Activ-12 results sterile and does not convey growing biota under the assayed conditions. | | | | |
| -The pH values corresponding to those conferred by most C-Activ-12 dilutions are themselves inhibitory to the growth of *P.synrigae* pv*.actinidiae.* This species showed a particular sensitivity to pH/HCl values below neutrality. It is worth noticing that at a pH as low as 6.56 there is already a clear growth inhibition. | | | | |
| -C-Activ-12 is nevertheless shown to be active also when the pH is neutralized by NaOH indicating that the growth inhibition is not due to acidity but to a genuine antagonistic mechanism exerted by its ingredients. | | | | |

**Bacteriocidal Efficiency Assay** - Besides determining the MIC, we assessed the capability of C-Activ-12 to permanently reduce viability of *P.syringae* pv. *actinidiae* upon contact with it at the 0.5% concentration. The abatement of colony forming units on plates was measured as follows:
Mezzo nutritivo: NB + 5% Sucrose (10 ml); Pseudomonas syringae pv. actinidiae ceppo 8.43 (inoculo 10 µl in 10 ml) ; Sostanza da saggiare: C-Activ-12
   - 5 µl of C-Activ-12 were added to 1 ml of exponential phase liquid pre-culture of *P. syringae* pv. Actinidiae strain 8.43 (1:200 dilution → yielding a 0.5% C-Activ-12 concentration)
   - The suspension was mixed and incubated 5 minutes at 20°C.
   - 100 µl of such suspension were transferred in a 50 ml sterile falcon tube and diluted to 10 ml with sterile saline solution, causing a dilution of C-Activ-12 of 1:20000 which is far beyond its MIC range. Serial dilutions were carried out in sterile saline solution and plated on NSA plates (Nutrient Agar with 5% Sucrose). A parallel test with the same cells was run without adding C-Activ-12. The CFU counts between control and C-Activ-12 treated cells were compared.

### Results: - Control : 0.7 x 10⁷ CFU/ml - Exposed for 5 min to 0.5% C-Activ-12: 1.6 x 10⁵ CFU/ml

Comments: - The treatment, with this exposure time and concentration, thus proved able to cause a 43-fold reduction in the titre of culturable cells, corresponding to an about **1.5** log reduction.

## Claims

1. A non-microbial non-toxic biocidal composition, having a pH in the range of from about 1 to 2.9, comprising:
from about 0.1% to about 15% w/w of at least one water soluble flavonoid compound, optionally in the form of an aqueous plant part extract comprising at least one flavonoid;
from about 8% w/w to about 12% w/w of at least one organic acid;
from about 30% w/w to about 50% w/w of at least one polyol; and
from about 5% w/w to about 10% w/w of at least one stablizer; and
the remainder of the composition is water.

2. The composition of claim 1 wherein the composition is diluted with water/organic acid solution, and comprises:
from about 0.01% to about 1.5% w/w of at least one water soluble flavonoid compound, optionally in the form of an aqueous plant part extract comprising at least one flavonoid;
from about 11.5% w/w to about 22.5% w/w of a first organic acid;
from about 1.5% w/w to about 2.5% w/w of a second organic acid;
from about 1% w/w to about 1.5% w/w of at least one polyol; and
from about 0.5% w/w to about 1% w/w of at least one stablizer; and
the remainder of the composition is water.

3. The composition of claim 1 or 2 wherein the at least one flavonoid is selected from poncirin, neoeriocitrin, isonaringin, rhoiflin, naringen, neodiosmin, hesperidin, neohesperidin or naringenin, wherein less than about 75% of the total flavonoid content are naringen and neohesperidin.

4. The composition of any preceding claim wherein in excess of about 15% of the total flavonoid content is isonaringin.

5. The composition of any preceding claim wherein the at least one flavonoid compound is in the form of an aqueous plant part extract comprising at least one flavonoid obtained from an aqueous extract of the pulp of fruit, the leaves, the flowers and/or the seeds of citrus aurantium/bitter oranges, Seville oranges, Citrus paradise and/or grapefruit.

6. The composition of any preceding claim wherein the at least one organic acid is a fruit acid selected from: lactic acids, acetic acids, malic acids or citric acids, or mixtures thereof, and/or wherein the stabliser may be selected from: ascorbic acid, uric acid or polyphenol.

7. The composition of claim 1, wherein the at least one organic acid is a mixture of malic acid and citric acid, and wherein the stabilizer is ascorbic acid, and wherein the ratio of stabilizer to organic acid in the composition is in the range of from about 1:2:2 (stabilizer: organic acid: organic acid) to about 2.5:3.2:3.2 (stabilizer: organic acid: organic acid).

8. The composition of claim 1 having
| **Component** | % w/w of total composition |
|---|---|
| water | 31 |
| Polyol | 21.5 |
| Stabiliser | 7.5 |
| Citric Acid | 17.5 |
| Malic Acid | 17.5 |
| Flavonoid Extract | 5 |

9. The composition of claim 2 having
| **Component** | % w/w of total composition |
|---|---|
| RO water | 83.1 |
| Polyol | 2.15 |
| Stabiliser | 0.75 |
| Citric Acid | 11.75 |
| Malic Acid | 1.75 |
| Flavonoid Extract | 0.5 |

10. The composition of any preceding claim further comprising at least one additional component or adjuvant selected from surfactants, for example SDS, alcohols, coatings, or bleach, or at least one acceptable carrier suitable for use in food and water applications.

11. Use of the composition according to any one of claim 1 to 10 as a biocide, as a sanitiser, in human or animal hygiene products/biocidal products, veterinary hygiene biocidal, in protective coatings, in surface sanitisation, in drinking water for poultry, in potable water treatment, in non-rinse surface sanitisers, in food processing aids, as an antimicrobial or as a disinfectant against microbes, planktonics, fungi, fungal spores or biofilms, as a food sanitiser or a wash for food produce such as salads, vegetables, fruit and plants, poultry and meat carcasses, as a barrier protectant, as a poultry processing aid such as a wash or spray, a drinking water additive for animals, surface sterilizers or disinfectant for surface and equipment, in packaging for storage for extension of shelf life & growth inhibition, in biosecurity & environmental management of microorganisms or in private area and public health areas disinfectants, in food and feed area disinfectants, as a drinking water disinfectant for human and animal consumption, in preservative applications such as preservatives for food or feedstocks, in-can preservatives, film preservatives/packaging, polymerised materials preservatives, preservatives for liquid-cooling and processing systems, crop and plant protection product, and in wounds and infections and dressings or bandages for same, or in a formulation for an aerosol dispenser, or with other liquids for disinfection, in the form of encapsulated granule, capsule suspension, emulsifiable concentrates, emulsions for seed treatment, flowable concentrate for seed treatment, cold or hot fogging concentrate, solution for seed treatment, suspension/flowable concentrate or soluble concentrate.

12. Use of the composition according to any one of claim 1 to 9 against gram positive and/or gram negative bacteria. Activity is against at least one bacterial species selected from the group consisting of: *Salmonella,* Campylobacter, *Enterococcus, Listeria monocytogenes, Pseudomonas, Escherichia* and *Stapylococcus* and *Pseudomonas.* In a preferred embodiment the biocide is used against *at least one bacterial strain* selected from the group consisiting of: *Salmonella typhimurium, Campylobacter jejuni, Enterococcus hirae, Listeria monocytogenes, Pseudomonas aeruginosa, Escherichia coli* and *Stapylococcus aureus, C. sakazakii, S. pollorum, P. cangingivalis, P. Salivosa, F. nucleatum, Eikenella corrodens, Bacteroides fragilis, Prevotella intermedia, Porphyromonas gingivalis, Tanerrella forsythensis, Cronobacter sakazakii,* fungi, powdery mildew, botrytis or penicillium.

13. A process for preparing biocidal composition comprising the steps of:
(i) dissolving at least one polyol in water to form a water/polyol co-solvent solution;
(ii) adding at least one organic acid to the water/polyol co-solvent solution, adding each organic acid sequentially, if more than one organic acid is used;
(iii) adding at least one stabiliser to the water/polyol co-solvent solution; and
(v) adding at least one water soluble flavonoid or at least one aqueous plant and/or fruit flavonoid extract, to form a non-microbe non-toxic biocide concentrate.

14. The process of claim 1 wherein the steps are carried out sequentially in the order described at a range of from about 45°C to about 55°C and the components are added in concentrations to provide a composition with pH from about 1.0 to about 2.0.

15. The process of claim 1 further comprising the steps of:
(iv) dissolving at least one organic acid in water to form a 10% w/w solution of organic acid;
(v) dissolving a biocidal composition obtained by the process of claim 13 into the solution formed in step (i);
(iii) diluting the solution formed in step (ii) with water,
to form an active working solution of a non-microbial non toxic biocidal composition comprising a 1/10 concentration of the biocidal composition obtained by the process of claim 13.
